(19) Europäisches Patentamt
European Patent Office
Office européen des brevets

(11) EP 1 241 168 A1

(12) DEMANDE DE BREVET EUROPEEN

(43) Date de publication:
**18.09.2002 Bulletin 2002/38**

(21) Numéro de dépôt: **02010824.7**

(22) Date de dépôt: **13.09.1996**

(51) Int Cl.[7]: **C07D 401/14**, C07D 401/06, C07D 417/14, A61K 31/445, C07D 211/58, C07D 211/52 // (C07D417/14, 277:00, 211:00, 211:00), (C07D417/14, 271:00, 211:00, 211:00)

(84) Etats contractants désignés:
**AT BE CH DE DK ES FI FR GB GR IE IT LI LU MC NL PT SE**

(30) Priorité: **14.09.1995 FR 9510776**

(62) Numéro(s) de document de la (des) demande(s) initiale(s) en application de l'article 76 CBE:
**96931126.5 / 1 019 373**

(71) Demandeur: **SANOFI-SYNTHELABO**
**75013 Paris (FR)**

(72) Inventeurs:
- **Bichon, Daniel**
  **34009 Montpellier (FR)**
- **Edmonds-Alt, Xavier**
  **34980 Combaillaux (FR)**
- **Gueule, Patrick**
  **34820 Teyran (FR)**
- **Proietto, Vincenzo**
  **34680 Saint-Georges-d'Orques (FR)**
- **Van Broeck, Didier**
  **34570 Murviel-les-Montpellier (FR)**

(74) Mandataire: **Gillard, Marie-Louise et al**
**Cabinet Beau de Loménie,**
**158, rue de l'Université**
**75007 Paris (FR)**

Remarques:
Cette demande a été déposée le 15 - 05 - 2002 comme demande divisionnaire de la demande mentionnée sous le code INID 62.

(54) **Composés antagonistes sélectifs du récepteur NK3 humain, procédé pour leur obtention et compositions pharmaceutiques les contenant**

(57) L'invention a pour objet des composés de formule (Ia)

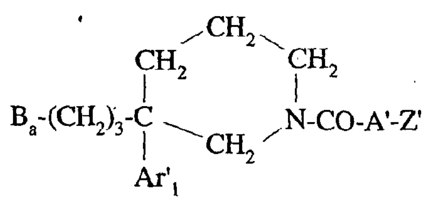

(Ia)

ainsi que leur procédé d'obtention et les compositions pharmaceutiques les contenant. Application : antagonistes sélectifs du récepteur $NK_3$ humain.

EP 1 241 168 A1

## Description

La présente invention a pour objet des nouveaux composés antagonistes sélectifs du récepteur $NK_3$ humain pour la préparation de médicaments utiles dans le traitement de maladies psychiatriques, de maladies d'origine psychosomatique, de l'hypertension et d'une manière générale de toute pathologie centrale ou périphérique dans laquelle la neurokinine B et le récepteur $NK_3$ interviennent dans les régulations interneuronales, un procédé pour leur obtention et les compositions pharmaceutiques en contenant en tant que principe actif.

Par maladie d'origine psychosomatique, on désigne, des maladies ayant leur origine dans le système nerveux central (SNC) et des conséquences pathologiques au niveau périphérique.

Dans les dernières années, de nombreux travaux de recherche ont été effectués sur les tachykinines et leurs récepteurs. Les tachykinines sont distribuées à la fois dans le système nerveux central et dans le système nerveux périphérique. Les récepteurs aux tachykinines ont été reconnus et sont classés en trois types : $NK_1$, $NK_2$, $NK_3$. La substance P (SP) est le ligand endogène des récepteurs $NK_1$, la neurokinine A ($NK_A$) celui des récepteurs $NK_2$ et la neurokinine B ($NK_B$), celui des récepteurs $NK_3$.

Les récepteurs $NK_1$, $NK_2$, $NK_3$ ont été mis en évidence chez différentes espèces. Ainsi, les récepteurs $NK_3$ ont été identifiés chez le cobaye, le rat, le singe (Br. J. Pharmacol., 1990, 99, 767-773 ; Neurochem. Int., 1991, 18, 149-165) ; ils ont également été mis en évidence chez l'homme (FEBS Letters, 1992, 299 (1), 90-95).

Une revue de C.A. Maggi et al. fait le point sur les récepteurs aux tachykinines et leurs antagonistes et expose les études pharmacologiques et les applications en thérapeutique humaine (J. Autonomic Pharmacol., 1993, 13, 23-93).

Parmi les antagonistes spécifiques du récepteur $NK_1$ on peut citer les composés non peptidiques suivants : CP-96345 (J. Med. Chem., 1992, 35, 2591-2600), RP-68651 (Proc. Natl. Acad. Sci. USA, 1991, 88, 10208-10212), SR 140333 (Curr. J. Pharmacol., 1993, 250, 403-413).

Pour le récepteur $NK_2$, un antagoniste sélectif non peptidique, le SR 48968 a été décrit en détail (Life Sci., 1992, 50, PL101-PL106).

En ce qui concerne le récepteur $NK_3$ humain, un antagoniste sélectif non peptidique, le chlorhydrate de (+) -N-[1-[3-[1-benzoyl-3-(3,4-dichlorophényl) pipérid-3-yl]propyl]-4-phénylpipérid-4-yl]-N-méthylacétamide ou SR 142801 a été décrit (EP-A-0673 928 ; Peptides and their antagonists in tissue injury, Montreal, Canada, 1994, July 31-August 3. Canadian J. Physiol. Pharmacol., 1994, 72 (suppl. 2), 25, Abst. III. 0. 9. ; Life Sci., 1994, 56 (1), 27-32 ; British Pharmacol. Society, Canterbury, 1995, April 6-8 ; Eur. J. Pharmacol., 1995, 278 (1), 17-25 ; 1st. Eur. Congress Pharmacol., Milan, 1995, June 16-19).

Les demandes de brevet EP 474 561 et EP 512901 décrivent, des antagonistes des neurokinines, plus particulièrement des antagonistes des récepteurs $NK_1$ ou $NK_2$. Les études pharmacologiques des antagonistes peptidiques et non-peptiques des récepteurs $NK_1$ et $NK_2$ ont montré que leurs affinités pour ces récepteurs ainsi que leurs activités pharmacologiques étaient très fortement fonction de l'espèce, très probablement suite à de faibles différences dans les séquences d'acides aminés, induisant ainsi de très fines variations structurales de ces récepteurs d'une espèce à l'autre (J. Autonomic Pharmacol., 1993, 13, 23-93). Certaines données expérimentales, confirmées par la caractérisation pharmacologique des composés objets de la présente invention, semblent indiquer qu'une situation comparable existe pour le récepteur $NK_3$. En particulier, le récepteur $NK_3$ humain se distingue du récepteur $NK_3$ du rat.

On a maintenant trouvé des composés non peptidiques qui présentent une très forte affinité pour le récepteur $NK_3$ humain et une grande spécificité pour ledit récepteur. Ces composés peuvent être utilisés pour la préparation de médicaments utiles dans le traitement de maladies psychiatriques ou d'origine psychosomatique et de toutes maladies centrales ou périphériques dans lesquelles la neurokinine B et le récepteur $NK_3$ interviennent dans les régulations interneuronales.

Par très forte affinité pour le récepteur $NK_3$ humain on entend une affinité caractérisée par une constante d'inhibition Ki généralement inférieure à $5.10^{-9}$M.

Dans les études de fixation d'un ligand, la constante d'inhibition Ki est définie par la relation de Cheng-Prusoff (in Receptor Binding in Drug Research, eds. R.A. O'BRIEN. Marcel Dekker, New York, 1986) :

$$Ki= \frac{IC_{50}}{1 + \frac{[L]}{Kd}}$$

[L] : concentration du ligand,
Kd : constante de dissociation du ligand,
$IC_{50}$ : concentration qui inhibe 50 % de la fixation du ligand.

Par grande spécificité pour le récepteur $NK_3$ humain, on entend que la constante d'inhibition (Ki) pour le récepteur $NK_3$ humain est généralement au moins 100 fois inférieure à la constante d'inhibition (Ki) pour le récepteur $NK_2$ ou à celle pour le récepteur $NK_1$ de différentes espèces.

Ainsi selon un de ses aspects, la présente invention a pour objet des composés de formule :

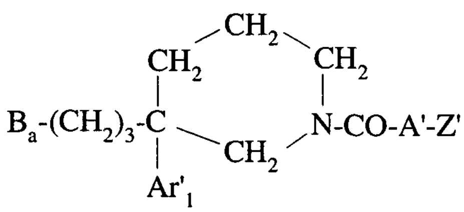

(Ia)

dans laquelle :

- $Ar'_1$ représente un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, un méthylènedioxy, lesdits substituants étant identiques ou différents ;
- A' représente une liaison directe ou un groupe $-CH_2-$ ;
- Z' représente :
  - . un phényle non substitué ou substitué une ou plusieurs fois par un substituant choisi parmi : un atome d'halogène ; un trifluorométhyle ; un cyano ; un hydroxy ; un nitro ; un amino non substitué ou substitué une ou deux fois par un $(C_1-C_4)$alkyle ; un benzylamino ; un carboxy ; un $(C_1-C_{10})$alkyle ; un $(C_3-C_8)$cycloalkyle non substitué ou substitué une ou plusieurs fois par un méthyle ; un $(C_1-C_{10})$alcoxy ; un $(C_3-C_8)$cycloalkyloxy non substitué ou substitué une ou plusieurs fois par un méthyle ; un mercapto ; un $(C_1-C_{10})$alkylthio ; un formyloxy ; un $(C_1-C_6)$alkylcarbonyloxy ; un formylamino ; un $(C_1-C_6)$ alkylcarbonylamino ; un benzoylamino ; un $(C_1-C_4)$alcoxycarbonyle ; un $(C_3-C_7)$ cycloalkyloxycarbonyle ; un carbamoyle non substitué ou substitué une ou deux fois par un $(C_1-C_4)$alkyle ; un uréido non substitué ou substitué une ou deux fois en position 3 par un $(C_1-C_4)$alkyle ou un $(C_3-C_7)$cycloalkyle ; un (pyrrolidin-1-yl)carbonylamino, lesdits substituants étant identiques ou différents ;
  - . un naphtyle non substitué ou substitué une ou plusieurs fois par un halogène, un trifluorométhyle, un $(C_1-C_4)$ alkyle, un hydroxy, un $(C_1-C_4)$alcoxy ;
  - . un pyridyle; un thiényle; un indolyle ; un quinolyle ; un benzothiényle ; un imidazolyle ;

- $B_a$ représente un groupe $B_{1a}$ de formule :

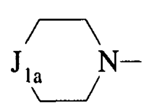

dans laquelle $J_{1a}$ représente un groupe

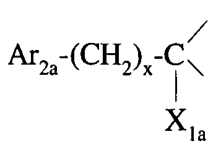

dans lequel :

- x est zéro ;
- $Ar_{2a}$ représente un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, un méthylènedioxy, lesdits substituants étant identiques ou différents ;

- $X_{1a}$ représente un groupe choisi parmi :

  . hydrogène ;
  . $(C_1-C_7)$alkyle ;
  . $-(CH_2)_m-OR_4$ dans lequel m est deux et $R_4$ représente un hydrogène ou un $(C_1-C_7)$alkyle ;
  . $-(CH_2)_m-OCOR_5$ dans lequel :

    m est deux et $R_5$ représente un hydrogène ; un $(C_1-C_7)$alkyle ; un $(C_3-C_7)$cycloalkyle non substitué ou substitué par un ou plusieurs méthyles ; un phényle ; un pyridyle ;ou
    m est zéro ou un et $R_5$ représente un $(C_3-C_7)$cycloalkyle non substitué ou substitué par un ou plusieurs méthyles ; un phényle ; un pyridyle ;

  . $-(CH_2)_m-OCONH(C_1-C_7)$alkyle dans lequel m est zéro ou deux ;
  . $-O-CH_2-CH_2-OR_6$ dans lequel $R_6$ représente un hydrogène ; un $(C_1-C_7)$alkyle; un formyle ; un $(C_1-C_7)$alkylcarbonyle ;
  . $-(CH_2)_n-SR_7$ dans lequel n est zéro ou un et $R_7$ représente un hydrogène ou un $(C_1-C_7)$alkyle ;
  . $-CH_2-S(O)_j-(C_1-C_7)$alkyle dans lequel j est un ou deux ;
  . $-NR_8R_9$ dans lequel $R_8$ et $R_9$ ensemble avec l'atome d'azote auquel ils sont liés constituent l'hétérocycle pipérazine non substitué ou substitué en position 4 par un $(C_1-C_4)$alkyle ;
  . $-(CH_2)_p-NR_{10}R_{11}$ dans lequel p est deux et $R_{10}$ et $R_{11}$ représentent chacun indépendamment un hydrogène ou un $(C_1-C_7)$alkyle ; $R_{11}$ peut de plus représenter un $(C_3-C_7)$cycloalkylméthyle ou un benzyle ;
  . $-NR_{12}COR_{13}$ dans lequel $R_{12}$ représente un hydrogène ou un $(C_1-C_7)$alkyle et $R_{13}$ représente un vinyle, un furyle, un thiényle, un pyrrolyle ou un imidazolyle;
  . $-NR_{14}COCOR_{15}$ dans lequel $R_{14}$ représente un hydrogène ou un $(C_1-C_7)$alkyle et $R_{15}$ représente un $(C_1-C_4)$alcoxy ;
  . $-(CH_2)_p-NR_{14}C(=W_1)R_{16}$ dans lequel p est deux, $W_1$ représente un atome d'oxygène ou un atome de soufre, $R_{14}$ représente un hydrogène ou un $(C_1-C_7)$alkyle et $R_{16}$ représente un hydrogène, un $(C_1-C_7)$alkyle ; un $(C_3-C_7)$cycloalkyle non substitué ou substitué par un ou plusieurs méthyles ; un phényle ; un benzyle ; un vinyle ; un pyridyle ; un furyle ; un thiényle ; un pyrrolyle ; un imidazolyle ; ou p est un, $W_1$ représente un atome de soufre et $R_{14}$ et $R_{16}$ sont tels que l'on vient de les définir ou bien $W_1$ représente un atome d'oxygène, $R_{14}$ est tel que l'on vient de le définir et $R_{16}$ représente un vinyle ; un furyle, un thiényle, un pyrrolyle ou un imidazolyle ;
  . $-(CH_2)_m-NR_{14}COOR_{17}$ dans lequel m est deux, $R_{14}$ représente un hydrogène ou un $(C_1-C_7)$alkyle et $R_{17}$ représente un $(C_1-C_7)$alkyle ou un phényle ;
  . $-(CH_2)_m-NR_{14}SO_2R_{18}$ dans lequel m est deux, $R_{14}$ représente un hydrogène ou un $(C_1-C_7)$alkyle et $R_{18}$ représente un $(C_1-C_7)$alkyle ; un amino libre ou substitué par un ou deux $(C_1-C_7)$alkyles ; un phényle non substitué ou substitué une ou plusieurs fois par un substituant choisi parmi : un atome d'halogène, un $(C_1-C_7)$alkyle, un trifluorométhyle, un hydroxy, un $(C_1-C_7)$alcoxy, un carboxy, un $(C_1-C_7)$alkylcarbonyloxy, un cyano, un nitro, un amino libre ou substitué par un ou deux $(C_1-C_7)$alkyles, lesdits substituants étant identiques ou différents ;
  . $-(CH_2)_m-NR_{14}C(=W_1)NR_{19}R_{20}$ dans lequel m est deux, $W_1$ représente un atome d'oxygène ou un atome de soufre, $R_{14}$ représente un hydrogène ou un $(C_1-C_7)$alkyle et $R_{19}$ et $R_{20}$ représentent chacun indépendamment un hydrogène ou un $(C_1-C_7)$alkyle ; $R_{20}$ peut de plus représenter un $(C_3-C_7)$cycloalkyle ; un $(C_3-C_7)$cycloalkylméthyle ; un hydroxy ; un $(C_1-C_4)$alcoxy ; un benzyle ; un phényle ; un $(C_1-C_7)$alkyle substitué par un hydroxy, un $(C_1-C_3)$alcoxy, un phényle, un carboxy, un $(C_1-C_3)$alcoxycarbonyle ou un carbamoyle non substitué ou substitué par un ou deux $(C_1-C_7)$alkyles ; ou bien $R_{19}$ et $R_{20}$ ensemble avec l'atome d'azote auquel ils sont liés constituent un hétérocycle choisi parmi : l'azétidine, la pyrrolidine, la pipéridine, la morpholine, la thiomorpholine, la perhydroazépine ou la pipérazine non substituée ou substituée en position 4 par un $(C_1-C_4)$alkyle ; ou m est zéro ou un, $W_1$ représente un atome de soufre et $R_{14}$, $R_{19}$ et $R_{20}$ sont tels que l'on vient de les définir ou bien $W_1$ représente un atome d'oxygène, $R_{14}$ et $R_{19}$ représentent chacun indépendamment un hydrogène ou un $(C_1-C_7)$alkyle et $R_{20}$ représente un $(C_1-C_7)$alkyle substitué par un hydroxy, un $(C_1-C_3)$alcoxy, un phényle, un carboxy, un $(C_1-C_3)$alcoxycarbonyle ou un carbamoyle non substitué ou substitué par un ou deux $(C_1-C_7)$alkyles ; ou bien $R_{19}$ et $R_{20}$ ensemble avec l'atome d'azote auquel ils sont liés constituent l'hétérocycle pipérazine non substitué ou substitué en position 4 par un $(C_1-C_4)$alkyle ;
  . $-(CH_2)_n-COOR_{21}$ dans lequel n est un et $R_{21}$ représente un hydrogène ou un $(C_1-C_7)$alkyle ; ou n est zéro et $R_{21}$ représente un hydrogène ;
  . $-(CH_2)_n-C(=W_1)NR_{19}R_{20}$ dans lequel n est un, $W_1$ représente un atome d'oxygène ou un atome de soufre et $R_{19}$ et $R_{20}$ représentent chacun indépendamment un hydrogène ou un $(C_1-C_7)$alkyle ; $R_{20}$ peut de plus repré-

senter un ($C_3$-$C_7$)cycloalkyle ; un ($C_3$-$C_7$)cycloalkylméthyle ; un hydroxy ; un ($C_1$-$C_4$)alcoxy ; un benzyle ; un phényle ; un ($C_1$-$C_7$)alkyle substitué par un hydroxy, un ($C_1$-$C_3$)alcoxy, un phényle, un carboxy, un ($C_1$-$C_3$) alcoxycarbonyle ou un carbamoyle non substitué ou substitué par un ou deux ($C_1$-$C_7$)alkyles ; ou bien $R_{19}$ et $R_{20}$ ensemble avec l'atome d'azote auquel ils sont liés constituent un hétérocycle choisi parmi : l'azétidine, la pyrrolidine, la pipéridine, la morpholine, la thiomorpholine, la perhydroazépine ou la pipérazine non substituée ou substituée en position 4 par un ($C_1$-$C_4$)alkyle ; ou n est zéro; $W_1$ représente un atome de soufre et $R_{19}$ et $R_{20}$ sont tels que l'on vient de les définir ou bien $W_1$ représente un atome d'oxygène, $R_{19}$ représente un hydrogène ou un ($C_1$-$C_7$)alkyle et $R_{20}$ représente un ($C_1$-$C_7$)alkyle substitué par un hydroxy, un ($C_1$-$C_3$)alcoxy, un phényle, un carboxy, un ($C_1$-$C_3$)alcoxycarbonyle ou un carbamoyle non substitué ou substitué par un ou deux ($C_1$-$C_7$)alkyles ; ou bien $R_{19}$ et $R_{20}$ ensemble avec l'atome d'azote auquel ils sont liés constituent l'hétérocycle pipérazine non substitué ou substitué en position 4 par un ($C_1$-$C_4$)alkyle ;

. -CO-$NR_{22}NR_{23}R_{24}$ dans lequel $R_{22}$ représente un hydrogène ou un ($C_1$-$C_7$)alkyle et $R_{23}$ et $R_{24}$ représentent chacun indépendamment un hydrogène ou un ($C_1$-$C_7$)alkyle ;

.

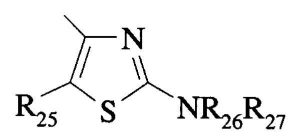

dans lequel $R_{25}$ représente un hydrogène ou un ($C_1$-$C_7$)alkyle, $R_{26}$ et $R_{27}$ représentent chacun indépendamment un hydrogène ou un ($C_1$-$C_7$)alkyle et $R_{27}$ peut de plus représenter un formyle ou un ($C_1$-$C_7$) alkylcarbonyle ;

.

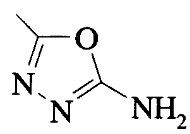 ;

ainsi que leurs sels et solvates, notamment pharmaceutiquement acceptables.

Les composés de formule (I) selon l'invention comprennent aussi bien les isomères optiquement purs que les racémiques.

On peut former des sels des composés de formule (I). Ces sels comprennent aussi bien ceux avec des acides minéraux ou organiques qui permettent une séparation ou une cristallisation convenable des composés de formule (I), tels que l'acide picrique ou l'acide oxalique ou un acide optiquement actif, par exemple un acide mandélique ou camphosulfonique, que ceux qui forment des sels pharmaceutiquement acceptables, tels que le chlorhydrate, le bromhydrate, le sulfate, l'hydrogénosulfate, le dihydrogénophosphate, le méthanesulfonate, le méthylsulfate, le maléate, le fumarate, le naphtalène-2 sulfonate, le glycolate, le gluconate, le citrate, l'iséthionate, le benzènesulfonate, le paratoluènesulfonate.

Dans la présente description les groupes alkyle ou alcoxy sont droits ou ramifiés ; par atome d'halogène on entend un atome de chlore, de brome, de fluor ou d'iode.

Parmi les composés de formule (Ia) ceux de formule :

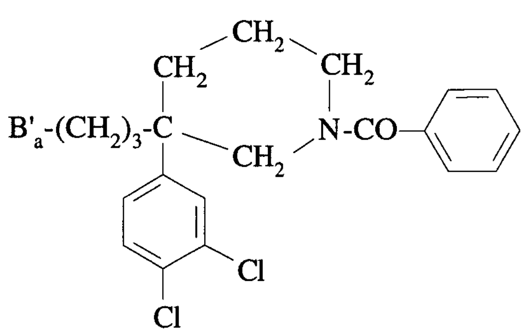

(I'a)

dans laquelle :

- $B'_a$ représente un groupe $B'_{1a}$ de formule :

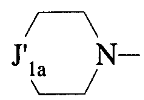

dans laquelle $J'_{1a}$ représente un groupe

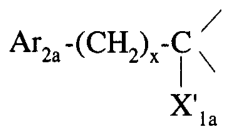

dans lequel :

- x est zéro ;
- $Ar_{2a}$ est tel que défini pour un composé de formule (Ia) ;
- $X'_{1a}$ représente un groupe choisi parmi :

  . $-O-CH_2-CH_2-OR_6$ dans lequel $R_6$ représente un hydrogène ; un $(C_1-C_7)$alkyle; un formyle ; un $(C_1-C_7)$alkylcarbonyle ;
  . $-NR_{12}COR_{13}$ dans lequel $R_{12}$ représente un hydrogène ou un $(C_1-C_7)$alkyle et $R_{13}$ représente un vinyle, un furyle, un thiényle, un pyrrolyle ou un imidazolyle ;
  . $-NR_{14}COCOR_{15}$ dans lequel $R_{14}$ représente un hydrogène ou un $(C_1-C_7)$alkyle et $R_{15}$ représente un $(C_1-C_4)$alcoxy ;
  . $-(CH_2)_p-NR_{14}C(=W_1)R_{16}$ dans lequel p est un, $W_1$ représente un atome d'oxygène, $R_{14}$ représente un hydrogène ou un $(C_1-C_7)$alkyle et $R_{16}$ représente un vinyle ; un furyle ; un thiényle ; un pyrrolyle ou un imidazolyle ;
  . $-(CH_2)_m-NR_{14}C(=W_1)NR_{19}R_{20}$ dans lequel m est zéro, $W_1$ représente un atome d'oxygène, $R_{14}$ représente un hydrogène ou un $(C_1-C_7)$alkyle, $R_{19}$ représente un hydrogène ou un $(C_1-C_7)$alkyle et $R_{20}$ représente un $(C_1-C_7)$alkyle substitué par un hydroxy, un $(C_1-C_3)$alcoxy, un phényle, un carboxy ; un $(C_1-C_3)$alcoxycarbonyle ou un carbamoyle non substitué ou substitué par un ou deux $(C_1-C_7)$alkyles ;
  . $-CO-NR_{22}-NR_{23}R_{24}$ dans lequel $R_{22}$ représente un hydrogène ou un $(C_1-C_7)$alkyle et $R_{23}$ et $R_{24}$ représentent chacun indépendamment un hydrogène ou un $(C_1-C_7)$alkyle ;
  .

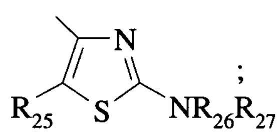

  dans lequel $R_{25}$ représente un hydrogène ou un $(C_1-C_7)$alkyle, $R_{26}$ et $R_{27}$ représentent chacun indépendamment un hydrogène ou un $(C_1-C_7)$alkyle et $R_{27}$ peut de plus représenter un formyle ou un $(C_1-C_7)$alkylcarbonyle ;
  .

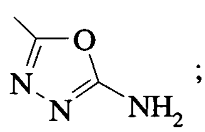

et leurs sels et solvates, notamment pharmaceutiquement acceptables, sont particulièrement préférés.

Parmi ces composés ceux de formule :

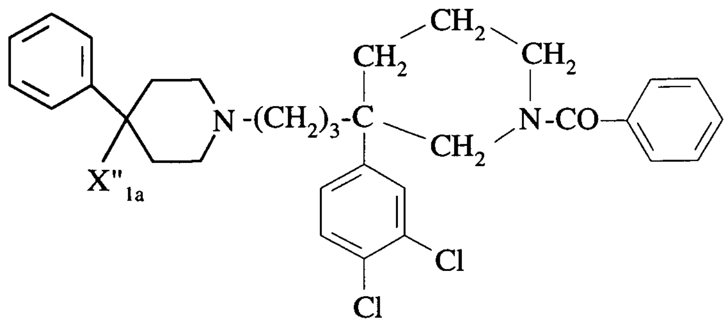

(I"a)

dans laquelle :

- X" $_{1a}$ représente un groupe choisi parmi :

  . $-O-CH_2-CH_2-OR_6$ dans lequel $R_6$ représente un hydrogène ; un $(C_1-C_7)$alkyle ; un formyle ; un $(C_1-C_7)$ alkylcarbonyle ; de préférence un hydrogène ou un acétyle ;
  . $-NR_{12}COR_{13}$ dans lequel $R_{12}$ représente un hydrogène ou $(C_1-C_7)$alkyle, de préférence un hydrogène, et $R_{13}$ représente un vinyle, un furyle, un thiényle, un pyrrolyle ou un imidazolyle, de préférence un furyle ou un thiényle ;
  . $-NR_{14}COCOR_{15}$ dans lequel $R_{14}$ représente un hydrogène ou un $(C_1-C_7)$alkyle, de préférence un hydrogène, et $R_{15}$ représente un $(C_1-C_4)$alcoxy, de préférence un éthoxy ;
  .

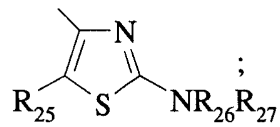

;

dans lequel $R_{25}$ représente un hydrogène ou un $(C_1-C_7)$alkyle, de préférence un hydrogène, et $R_{26}$ et $R_{27}$ représentent chacun indépendamment un hydrogène ou un $(C_1-C_7)$alkyle et $R_{27}$ peut de plus représenter un formyle ou un $(C_1-C_7)$alkylcarbonyle, de préférence $R_{26}$ et $R_{27}$ représentent un hydrogène ;

  .

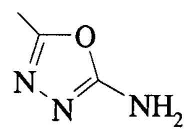

;

et leurs sels et solvates, notamment pharmaceutiquement acceptables, sont plus particulièrement préférés.

Les composés suivants :

la 3-[3-[4-(acryloyl-N-méthylamino)-4-phénylpipérid-1-yl]propyl-1-benzoyl-3-(3,4-dichlorophényl)pipéridine ;
la 3-[3-[4-(2-aminothiazol-4-yl)-4-phénylpipérid-1-yl]propyl]-1-benzoyl-3-(3,4-dichlorophényl)pipéridine ;
la 1-benzoyl-3-(3,4-dichlorophényl)-3-[3-[4-(2-hydroxyéthoxy)-4-phénylpipérid-1-yl]propyl]pipéridine ;
la 3-[3-[4-(2-acétyloxyéthoxy)-4-phénylpipérid-1-yl]propyl]-1-benzoyl-3-(3,4-dichlorophényl)pipéridine ;
la 1-benzoyl-3-(3,4-dichlorophényl)-3-[3-[4-(2-furoylamino)-4-phénylpipérid-1-yl]propyl]pipéridine ;
la 1-benzoyl-3-(3,4-dichlorophényl)-3-[3-[4-(2-thénoylamino)-4-phénylpipérid-1-yl]propyl]pipéridine ;
la 3-[3-[4-(2-amino-1,3,4-oxadiazol-5-yl)-4-phénylpipérid-1-yl]propyl]-1-benzoyl-3-(3,4-dichlorophényl) pipéridine ;
la 1-benzoyl-3-(3,4-dichlorophényl)-3-[3-[4-(éthoxalylamino)-4-phénylpipérid-1-yl]propyl]pipéridine ;

sous forme de racémates ou de l'un de leurs énantiomères (+) ou (-),
et leurs sels, notamment pharmaceutiquement acceptables sont tout particulièrement préférés, selon la présente invention.

L'invention concerne également lorsqu'ils existent, les solvates des composés de l'invention et de leurs sels, à savoir des composés de formules (Ia), (I'a) et (I"a) et des sels de ceux-ci.

Les composés selon l'invention sont obtenus par des méthodes connues en particulier celles qui sont décrites dans les demandes de brevet EP-A-474561 et EP-A-512901.

Un des procédés qui convient pour l'obtention des composés de formule (Ia) et de leurs sels est décrit ci-après.

Selon ce procédé :

1) on traite un composé de formule :

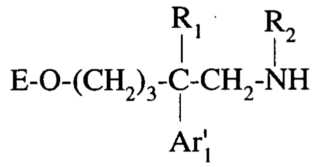

(II)

dans laquelle $Ar'_1$ est tel que défini pour un composé de formule (Ia), E représente l'hydrogène ou un groupe O-protecteur et $R_1$ et $R_2$ forment le groupe $-(CH_2)_3-$ avec un dérivé fonctionnel d'un acide de formule :

HO-CO-A'-Z' (IIIa)

dans laquelle A' et Z' sont tels que définis pour un composé de formule (Ia), pour obtenir un composé de formule :

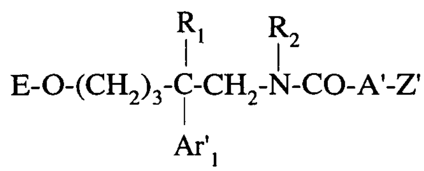

(IV)

2) on élimine éventuellement le groupe O-protecteur du composé de formule (IV), par action d'un acide ou d'une base, pour obtenir l'alcool de formule :

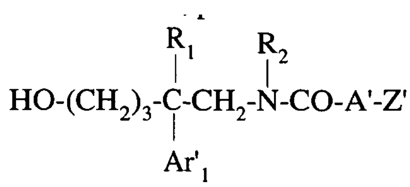

(V)

3) on traite l'alcool (V) avec un composé de formule :

$G-SO_2-Cl$ (VI)

dans laquelle G représente un groupe méthyle, phényle, tolyle ou trifluorométhyle, pour obtenir un composé de formule :

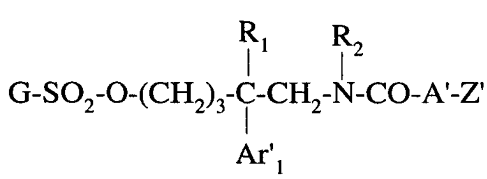

(VII)

4) on fait réagir le composé (VII)

avec une amine secondaire cyclique de formule :

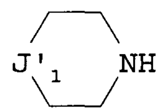 (VIIIa)

dans laquelle $J'_1$ représente :

un groupe

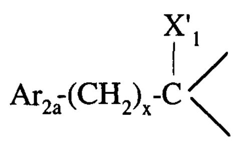

dans lequel $Ar_{2a}$ et x sont tels que définis pour (Ia) et $X'_1$ représente soit $X_{1a}$ tel que défini pour (Ia), soit un précurseur de $X_{1a}$, étant entendu que lorsque $X_{1a}$ contient un groupe hydroxyle ou un groupe aminé, ces groupes peuvent être protégés ;

5) et, après déprotection éventuelle des groupes hydroxyles ou des groupes aminés, ou transformation éventuelle de $X'_{1a}$ en $X_{1a}$, on transforme éventuellement le produit ainsi obtenu en l'un de ses sels avec un acide minéral ou organique.

Selon une variante du procédé :

1') on protège l'atome d'azote du composé de formule (II) pour obtenir un composé de formule :

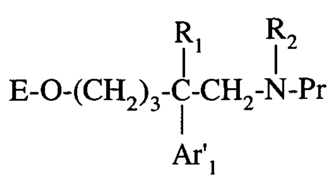 (XVII)

dans laquelle $Ar'_1$ est tel que défini pour un composé de formule (Ia), E représente l'hydrogène ou un groupe O-protecteur, Pr représente un groupe N-protecteur, tel que le groupe trityle, *tert*-butoxycarbonyle ou benzyloxycarbonyle, et $R_1$ et $R_2$ ensemble représentent le groupe $-(CH_2)_3-$,

2') on élimine éventuellement le groupe O-protecteur du composé de formule (XVII), par action d'un acide ou d'une base, pour obtenir l'alcool de formule :

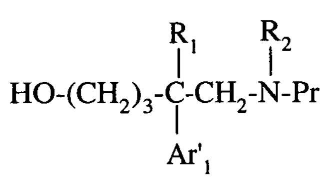 (XVIII)

3') on traite l'alcool (XVIII) avec un composé de formule (VI) tel que défini précédemment pour obtenir un composé de formule :

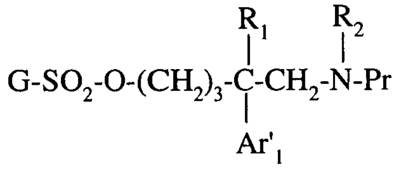 (XIX)

4') on fait réagir le composé (XIX) avec un composé de formule (VIIIa) tel que défini précédemment, pour obtenir un composé de formule :

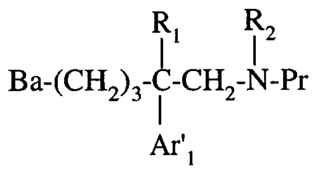

(XX)

dans laquelle Ba est tel que défini pour un composé de formule (Ia) étant entendu que lorsque Ba contient un groupe hydroxyle ou un groupe aminé, ces groupes peuvent être protégés ;

5') on élimine sélectivement le groupe protecteur Pr du composé de formule (XX), pour obtenir le composé de formule :

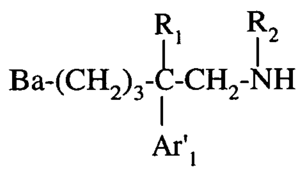

(XXI)

6') on traite le composé de formule (XXI) avec un composé de formule (IIIa), tel que défini précédemment ;

7') et, après déprotection éventuelle des groupes hydroxyles ou des groupes aminés, on transforme éventuellement le produit ainsi obtenu en l'un de ses sels avec un acide minéral ou organique.

Au cours de l'une quelconque des étapes de préparation des composés de formule (Ia) et plus particulièrement lorsqu'on met en oeuvre des composés de formule (VIIIa), ou des composés intermédiaires de formule (II), (IV), (XX), (XXI), il peut être nécessaire et/ou souhaitable de protéger les groupes fonctionnels réactifs ou sensibles, tels que les groupes amine, hydroxyle, ou carboxy, présents sur l'une quelconque des molécules concernées. Cette protection peut s'effectuer en utilisant les groupes protecteurs conventionnels, tels que ceux décrits dans Protective Groups in Organic Chemistry, J.F.W. McOmie, Ed. Plenum Press, 1973 et dans Protective Groups in Organic Synthesis, T.W. Greene et P.G.M. Wutts, Ed. John Wiley et Sons, 1991. L'élimination des groupes protecteurs peut s'effectuer à une étape ultérieure opportune en utilisant les méthodes connues de l'homme de l'art et qui n'affectent pas le reste de la molécule concernée.

Ainsi, lorsque E représente un groupe O-protecteur, celui-ci est choisi parmi les groupes O-protecteurs classiques bien connus de l'homme de l'art, tels que, par exemple, le tétrahydropyran-2-yle, le benzoyle ou un ($C_1$-$C_4$) alkylcarbonyle.

Les groupes O-protecteurs éventuellement utilisés pour obtenir un composé de formule (I) dans laquelle $X_{1a}$ contient un hydroxyle sont les groupes O-protecteurs classiques bien connus de l'homme de l'art tels que définis ci-dessus pour E.

Les groupes N-protecteurs éventuellement utilisés pour obtenir un composé de formule (I) dans laquelle $X_{1a}$ contient un groupe aminé sont les groupes N-protecteurs classiques bien connus de l'homme de l'art tels que, par exemple, le groupe trityle, méthoxytrityle, *tert*-butoxycarbonyle ou benzyloxycarbonyle.

Dans l'étape 1) ou dans l'étape 6') comme dérivé fonctionnel de l'acide (IIIa), on utilise l'acide lui-même, ou bien un des dérivés fonctionnels qui réagissent avec les amines, par exemple un anhydride, un anhydride mixte, le chlorure d'acide, ou un ester activé, comme l'ester de paranitrophényle.

Lorsqu'on met en oeuvre l'acide de formule (IIIa) lui-même, on opère en présence d'un agent de couplage utilisé en chimie peptidique tel que le 1,3-dicyclohexylcarbodiimide ou l'hexafluorophosphate de benzotriazol-1-yloxytris (diméthylamino)phosphonium en présence d'une base telle que la triéthylamine ou la N,N-diisopropyléthylamine, dans un solvant inerte tel que le dichlorométhane ou le N,N-diméthylformamide à une température comprise entre 0°C et la température ambiante.

Lorsqu'on utilise un chlorure d'acide, la réaction s'effectue dans un solvant inerte tel que le dichlorométhane ou le benzène, en présence d'une base telle que la triéthylamine ou la N-méthylmorpholine et à une température comprise entre -60°C et la température ambiante

Dans l'étape 2) du procédé ou dans l'étape 2') de la variante, éventuellement on déprotège le composé de formule (IV) ou le composé de formule (XVII) ainsi obtenu selon les méthodes connues de l'homme de l'art. Par exemple, lorsque E représente un groupe tétrahydropyran-2-yle, la déprotection s'effectue par hydrolyse acide en utilisant

l'acide chlorhydrique dans un solvant tel que l'éther, le méthanol ou le mélange de ces solvants, ou en utilisant le *p*-toluènesulfonate de pyridinium dans un solvant tel que le méthanol ou encore, en utilisant une résine Amberlyst® dans un solvant tel que le méthanol. La réaction s'effectue à une température comprise entre la température ambiante et la température de reflux du solvant. Lorsque E représente un groupe benzoyle ou un groupe ($C_1$-$C_4$)alkylcarbonyle, la déprotection s'effectue par hydrolyse en milieu alcalin en utilisant par exemple un hydroxyde de métal alcalin tel que l'hydroxyde de sodium, l'hydroxyde de potassium ou l'hydroxyde de lithium, dans un solvant inerte tel que l'eau, le méthanol, l'éthanol, le dioxane ou un mélange de ces solvants, à une température comprise entre 0°C et la température de reflux du solvant.

Dans l'étape 3) du procédé ou dans l'étape 3') de la variante, la réaction de l'alcool de formule (V) ou de l'alcool de formule (XVIII) avec un chlorure de sulfonyle de formule (VI) s'effectue en présence d'une base telle que la triéthylamine, la pyridine, la N,N-diisopropyléthylamine ou la N-méthylmorpholine, dans un solvant inerte tel que le dichlorométhane, le benzène ou le toluène et à une température comprise entre -20°C et la température de reflux du solvant.

Dans l'étape 4) ou dans l'étape 4'), le composé (VII) ou le composé (XIX) ainsi obtenu est mis en réaction avec un composé de formule (VIIIa). La réaction s'effectue dans un solvant inerte tel que le N,N-diméthylformamide, l'acétonitrile, le chlorure de méthylène, le toluène, l'isopropanol ou un mélange de ces solvants et en présence ou en l'absence d'une base. Lorsqu'on utilise une base, celle-ci est choisie parmi les bases organiques telles que la triéthylamine, la N,N-diisopropyléthylamine ou la N-méthylmorpholine ou parmi les carbonates ou bicarbonates de métal alcalin tels que le carbonate de potassium, le carbonate de sodium ou le bicarbonate de sodium. En l'absence de base, la réaction s'effectue en utilisant un excès du composé de formule (VIIIa), et éventuellement en présence d'un iodure de métal alcalin tel que l'iodure de potassium ou l'iodure de sodium. La réaction s'effectue à une température comprise entre la température ambiante et 100°C.

Dans l'étape 5') de la variante, on déprotège le composé de formule (XX) obtenu, selon les méthodes connues de l'homme de l'art.

On obtient finalement après déprotections éventuelles des groupes hydroxyles ou des groupes aminés, ou transformation éventuelle de $X'_1$ en $X_{1a}$ les composés de formule (Ia) selon l'invention.

Les composés de formule (Ia) sont isolés sous forme de base libre ou de sel selon les techniques classiques.

Ainsi lorsque le composé de formule (Ia) est obtenu sous forme de base libre, la salification est effectuée par traitement avec l'acide choisi dans un solvant organique. Par traitement de la base libre, dissoute par exemple dans un éther tel que l'éther diéthylique ou dans un alcool tel que le propan-2-ol ou dans l'acétone ou dans le dichlorométhane ou dans l'acétate d'éthyle, avec une solution de l'acide choisi dans le même solvant, on obtient le sel correspondant qui est isolé selon les techniques classiques.

Ainsi, on prépare par exemple le chlorhydrate, le bromhydrate, le sulfate, l'hydrogénosulfate, le dihydrogénophosphate, le méthanesulfonate, l'oxalate, le maléate, le fumarate, le naphtalène-2-sulfonate, le benzènesulfonate.

A la fin de la réaction, les composés de formule (Ia) peuvent être isolés sous forme d'un de leurs sels, par exemple le chlorhydrate ; dans ce cas, s'il est nécessaire, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

Les composés de formule (II) s'obtiennent par des méthodes connues en particulier celles qui sont décrites dans les demandes de brevet EP-A-0428434, EP-A-0474561, EP-A-0512901.

De façon particulière on peut préparer un composé de formule (II) dans laquelle $R_1$ et $R_2$ ensemble constituent un groupe $-(CH_2)_3-$ et E représente un hydrogène selon le SCHEMA 1 ci-après :

SCHEMA 1

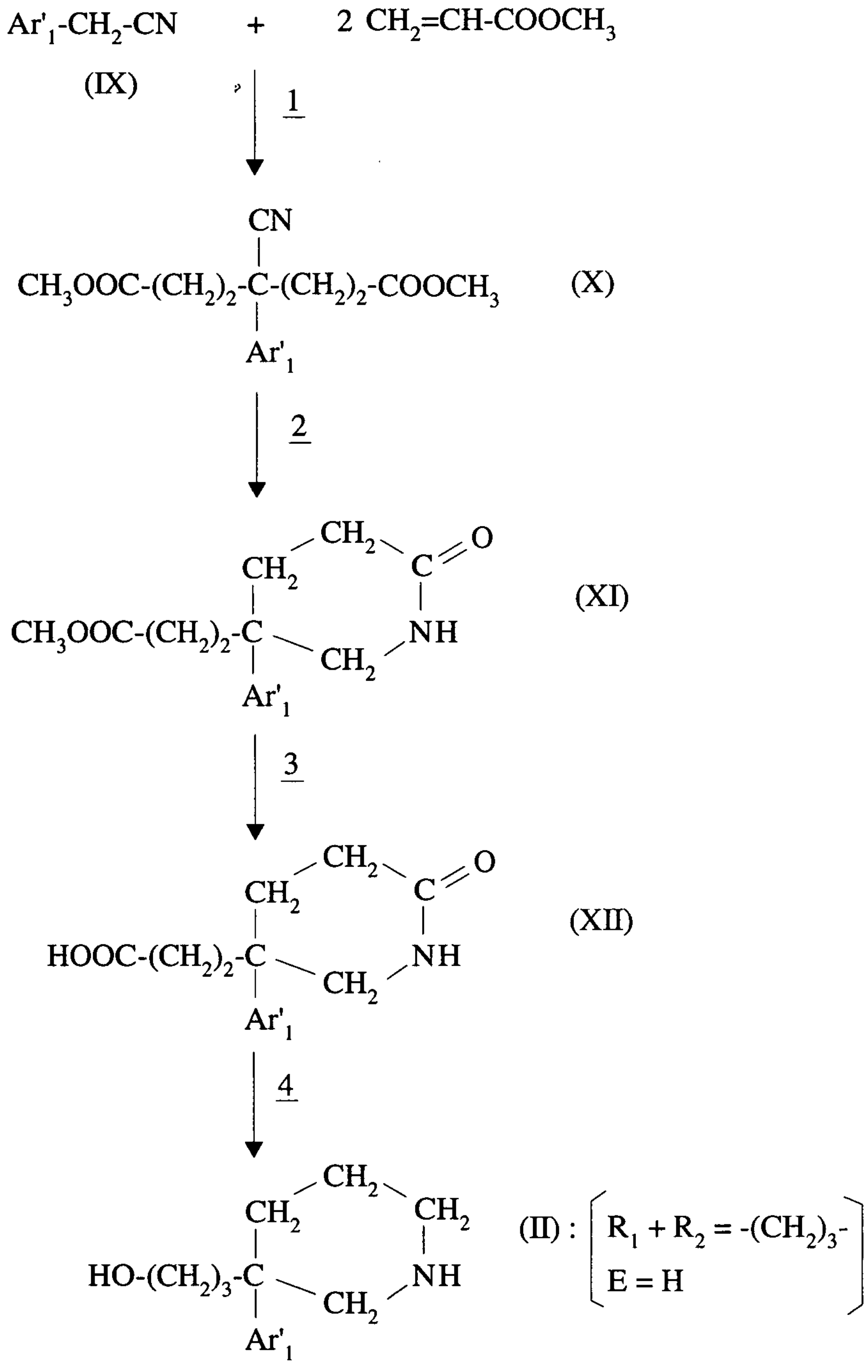


(IX) (X) (XI) (XII) (II) : $R_1 + R_2 = -(CH_2)_3-$ ; $E = H$

A l'étape 1, la réaction d'un composé de formule (IX) avec l'acrylate de méthyle en présence d'une base telle que le Triton®B ou le 1,8-diazabicyclo[5.4.0]undec-7-ène (DBU) permet d'obtenir le composé de formule (X). La réaction s'effectue dans un solvant inerte tel que le 1,4-dioxane ou le tétrahydrofurane et à une température comprise entre 60°C et la température de reflux du solvant.

A l'étape 2, le composé de formule (X) est soumis à une hydrogénation en présence d'un catalyseur tel que le nickel de Raney® pour obtenir le composé de formule (XI). La réaction s'effectue dans un solvant inerte tel qu'un alcanol, de préférence l'éthanol ou le 2-méthoxyéthanol, à une température comprise entre la température ambiante et 60°C et à une pression comprise entre la pression atmosphérique et 20 bars.

A l'étape 3, le composé de formule (XI) est soumis à une hydrolyse en milieu alcalin en utilisant par exemple une hydroxyde de métal alcalin tel que l'hydroxyde de sodium, ou l'hydroxyde de potassium dans un solvant tel que

l'eau, le méthanol ou un mélange de ces solvants et à une température comprise entre la température ambiante et la température de reflux du solvant.

Le composé de formule (XII) ainsi obtenu est réduit à l'étape 4 pour conduire au composé de formule (II) attendu. La réduction s'effectue au moyen d'un agent réducteur tel que l'hydrure d'aluminium et de lithium, l'hydrure de diisobutylaluminium, ou le borane dans le THF, dans un solvant inerte tel que le tétrahydrofurane, le 1,2-diméthoxyéthane ou le toluène à une température comprise entre la température ambiante et la température de reflux du solvant.

Les composés de formule (IIIa) sont connus ou préparés par des méthodes connues.

Les pipéridines de formule (Villa) sont connues ou préparées par des méthodes connues, telles que celles décrites dans EP-A-0428434, EP-A-0474561, EP-A-0512901 et EP-A-0515240.

On peut également préparer les pipéridines de formule (VIIIa) par des méthodes bien connues de l'homme de l'art, telles que celles décrites dans les publications suivantes :

J. Heterocyclic. Chem., 1986, 23, 73-75 ;
J. Chem. Soc., 1950, 1469 ;
J. Chem. Soc., 1945, 917 ;
J. Pharm. Sci., 1972, 61, 1316-1317 ;
J. Org. Chem., 1957, 22, 1484-1489 ;
Chem. Ber., 1975, 108, 3475-3482.

Les composés de formule (Villa) sont généralement préparés sous forme protégée sur l'azote de la pipéridine ; après une étape de déprotection, on obtient les composés de formule (VIIIa) eux-mêmes.

On indiquera ci-après, à titre d'exemples, différents procédés pour l'obtention des composés de formule (VIIIa) dans lesquelles, sauf stipulation contraire, les différents substituants sont tels que définis pour la formule (Ia).

Par ailleurs, on prépare un composé de formule (VIIIa) dans laquelle $X'_{1a}$ représente un groupe $-(CH_2)_m-OR_4$ dans lequel $R_4$ représente l'hydrogène et m est un ou respectivement deux, par réduction d'un composé de formule (VIIIa) dans laquelle $X'_{1a}$ représente un méthoxycarbonyle ou respectivement un méthoxycarbonylméthyle selon la méthode décrite dans Chem. Ber., 1975, 108, 3475-3482.

On peut également préparer un composé de formule (VIIIa) dans laquelle $X'_{1a}$ représente un groupe $-(CH_2)_m-OR_4$ dans lequel $R_4$ représente un $(C_1-C_7)$alkyle par alkylation d'un composé de formule (VIIIa) dans laquelle $X'_{1a}$ représente un groupe $-(CH_2)_m-OH$ selon les méthodes connues de l'homme de l'art.

On peut également préparer un composé de formule (VIIIa) dans laquelle $X'_{1a}$ représente un groupe $-O-CH_2-CH_2-OR_6$ dans lequel $R_6$ représente l'hydrogène par réaction d'un composé de formule (VIIIa) dans laquelle $X'_{1a}$ représente un benzoyloxy- avec l'éthylèneglycol en présence d'un acide tel que l'acide sulfurique.

Par une réaction identique et en utilisant un 2-$(C_1-C_7)$alcoxyéthanol, on prépare les composés de formule (VIIIa) dans laquelle $X'_{1a}$ représente un groupe $-O-CH_2CH_2-OR_6$ dans lequel $R_6$ représente un $(C_1-C_7)$alkyle.

Par action de l'acide formique sur un composé de formule (VIIIa) dans laquelle $X'_{1a}$ représente un groupe $-O-CH_2CH_2-OH$ on prépare les composés de formule (VIIIa) dans laquelle $X'_1$ représente un groupe $-O-CH_2CH_2-OR_6$ dans lequel $R_6$ représente un formyle. Par action d'un chlorure d'acide en $C_2-C_8$ et en présence d'une base telle que la triéthylamine, on prépare les composés de formule (VIIIa) dans laquelle $X'_1$ représente un groupe $-O-CH_2CH_2-OR_6$ dans lequel $R_6$ représente un $(C_1-C_7)$alkylcarbonyle.

Les composés de formule (VIIIa) dans laquelle $X'_{1a}$ représente un groupe $-(CH_2)_n-SR_7$ ou un groupe $-CH_2-S(O)_j-(C_1-C_7)$alkyle sont connus ou préparés par des méthodes connues, telles que celles décrites dans WO 95/12577.

Par action d'un chlorure d'acide $R_5COCl$ ($R_5$ différent de l'hydrogène) sur un composé de formule (VIIIa) dans laquelle $X'_{1a}$ représente un groupe $-(CH_2)_m-OH$ et en présence d'une base telle que la triéthylamine, on prépare les composés de formule (VIIIa) dans laquelle $X'_{1a}$ représente un groupe $-(CH_2)_m-OCOR_5$ ($R_5$ différent de l'hydrogène).

Par action de l'acide formique sur un composé de formule (VIIIa) dans laquelle $X'_{1a}$ représente un groupe $-(CH_2)_m-OH$, on prépare les composés de formule (VIIIa) dans laquelle $X'_{1a}$ représente un groupe $-(CH_2)_m-OCOR_5$ dans lequel $R_5$ représente l'hydrogène.

Par action d'un chlorure de carbamoyle $(C_1-C_7)$alkyl-NHCOCl sur les composés de formule (VIIIa) dans laquelle $X'_{1a}$ représente un groupe $-(CH_2)_m-OH$, on obtient les composés de formule (VIIIa) dans laquelle $X'_{1a}$ représente un groupe $(C_1-C_7)$alkyl-NHCOO-$(CH_2)_m$-. On prépare les mêmes composés par action d'un isocyanate $(C_1-C_7)$alkyl-N=C=O sur les composés de formule (Villa) dans laquelle $X'_{1a}$ représente un groupe $-(CH_2)_m-OH$.

Les composés de formule (VIIIa) dans laquelle $X'_{1a}$ représente un hydroxyle et qui portent un groupe protecteur sur l'azote de la pipéridine, peuvent subir une réaction de Ritter par action de l'acétonitrile pour préparer les composés de formule (VIIIa) dans laquelle $X'_{1a}$ est un acétamido. Par hydrolyse en milieu acide, on prépare ensuite les composés de formule (VIIIa) dans laquelle $X'_{1a}$ est un groupe $-NR_8R_9$ dans lequel $R_8$ et $R_9$ représentent chacun l'hydrogène.

On peut également préparer un composé de formule (VIIIa) dans laquelle $X'_{1a}$ représente un groupe $-NR_8R_9$ dans lequel $R_8$ et $R_9$ représentent chacun l'hydrogène par hydrolyse en milieu acide fort, par exemple l'acide chlorhydrique, d'un composé de formule (VIIIa) dans laquelle $X'_{1a}$ représente un groupe isocyanato.

Pour préparer un composé de formule (VIIIa) dans laquelle $X'_{1a}$ représente un groupe $-NR_8R_9$ dans lequel $R_8$ représente l'hydrogène et $R_9$ représente un $(C_1-C_7)$alkyle, ou respectivement un $(C_3-C_7)$cycloalkylméthyle ou un benzyle on peut effectuer une réduction d'un composé de formule (VIIIa) dans laquelle $X'_{1a}$ représente un groupe $-NR_{12}COR_{13}$ dans lequel $R_{12}$ représente l'hydrogène et $R_{13}$ représente un hydrogène ou un $(C_1-C_6)$alkyle, ou respectivement un $(C_3-C_7)$cycloalkyle ou un phényle. La réaction s'effectue au moyen d'un agent réducteur tel que l'hydrure d'aluminium et de lithium dans un solvant tel que le tétrahydrofurane à la température de reflux du solvant.

Par une réaction identique on peut préparer les composés de formule (VIIIa) dans laquelle $X'_{1a}$ représente un groupe $-NR_8R_9$ dans lequel $R_8$ représente un $(C_1-C_7)$alkyle à partir des composés de formule (VIIIa) dans laquelle $X'_{1a}$ représente un groupe $-NR_{12}COR_{13}$ dans lequel $R_{12}$ représente un $(C_1-C_7)$alkyle.

On prépare un composé de formule (VIIIa) dans laquelle $X'_{1a}$ représente un groupe $-NR_8R_9$ dans lequel $R_8$ et $R_9$ ensemble avec l'atome d'azote auquel ils sont liés constituent un hétérocycle, par application ou adaptation de la réaction de Bruylants (Bull. Soc. Chim. Belges, 1924, 33, 467 et Tetrahedron Letters, 1988, 29 (52), 6827-6830).

Pour préparer un composé de formule (VIIIa) dans laquelle $X'_{1a}$ représente un groupe $-CH_2-NR_{10}R_{11}$ dans lequel $R_{10}$ et $R_{11}$ représentent chacun l'hydrogène, on effectue la réduction d'un composé de formule (VIIIa) dans laquelle $X'_{1a}$ représente un cyano. Cette réduction s'effectue selon les méthodes bien connues de l'homme de l'art.

On prépare un composé de formule (VIIIa) dans laquelle $X'_{1a}$ représente un groupe $-CH_2-CH_2-NR_{10}R_{11}$ dans lequel $R_{10}$ et $R_{11}$ représentent chacun un hydrogène, à partir d'un composé de formule (VIIIa) dans laquelle $X'_{1a}$ représente un groupe $-CH_2-CH_2-OH$, par application ou adaptation de la méthode décrite dans J. Med. Chem., 1989, 32, 391-396.

On peut préparer les composés de formule (VIIIa) dans laquelle $X'_{1a}$ représente un groupe $-(CH_2)_p-NR_{10}R_{11}$ dans lequel $R_{10}$ représente un hydrogène ou un $(C_1-C_7)$alkyle et $R_{11}$ représente un $(C_1-C_7)$alkyle, un $(C_3-C_7)$cycloalkylméthyle ou un benzyle par réduction d'un composé de formule (VIIIa) dans laquelle $X'_{1a}$ représente un groupe $-(CH_2)_p-NR_{14}C(=W_1)R_{16}$ dans lequel $R_{14}$ représente un hydrogène ou un $(C_1-C_7)$alkyle, $R_{16}$ représente un hydrogène, un $(C_1-C_6)$alkyle, un $(C_3-C_7)$cycloalkyle ou un phényle et $W_1$ représente un atome d'oxygène.

Les composés de formule (VIIIa) dans laquelle $X'_{1a}$ représente un groupe $-NR_{12}COR_{13}$ dans lequel $R_{12}$ représente un hydrogène ou un $(C_1-C_7)$alkyle et $R_{13}$ représente l'hydrogène ou respectivement un $(C_1-C_7)$alkyle, un $(C_3-C_7)$cycloalkyle éventuellement substitué, un phényle, un benzyle, un vinyle, un pyridyle, un furyle, un thiényle, un pyrrolyle ou un imidazolyle, s'obtiennent par action de l'acide formique dans l'anhydride acétique ou respectivement d'un chlorure d'acide approprié $R_{13}COCl$, en présence d'une base telle que la triéthylamine, sur un composé de formule (VIIIa) dans laquelle $X'_{1a}$ représente un groupe $-NHR_{12}$. De façon particulière, on peut préparer un composé de formule (VIIIa) dans laquelle $X'_{1a}$ représente un groupe $-NR_{12}COR_{13}$ dans lequel $R_{13}$ représente un radical éthyle par hydrogénation, en présence d'un catalyseur tel que le palladium sur charbon, d'un composé de formule (VIIIa) dans laquelle $X'_{1a}$ représente un groupe acryloylamino ou acryloyl-N-$(C_1-C_7)$alkylamino.

On prépare un composé de formule (VIIIa) dans laquelle $X'_{1a}$ représente un groupe $-NR_{14}COCOR_{15}$ dans lequel $R_{15}$ représente un $(C_1-C_4)$alcoxy par réaction d'un composé de formule $Cl-COCOR_{15}$ avec un composé de formule (VIIIa) dans laquelle $X'_{1a}$ représente un groupe $-NHR_{14}$.

Les composés de formule (VIIIa) dans laquelle $X'_{1a}$ représente un groupe $-(CH_2)_p-NR_{14}C(=W_1)R_{16}$ dans lequel $W_1$ représente un atome d'oxygène, p est 1 ou 2, $R_{14}$ représente un hydrogène ou un $(C_1-C_7)$alkyle et $R_{16}$ représente un hydrogène ou respectivement, un $(C_1-C_7)$alkyle, un phényle, un benzyle, un pyridyle, un $(C_3-C_7)$cycloalkyle éventuellement substitué, un vinyle, un furyle, un thiényle, un pyrrolyle ou un imidazolyle, s'obtiennent par action, de l'acide formique dans l'anhydride acétique ou respectivement d'un chlorure d'acide approprié $R_{16}COCl$ en présence d'une base telle que la triéthylamine, sur un composé de formule (VIIIa) dans laquelle $X'_{1a}$ représente un groupe $-CH_2-NHR_{14}$ ou $-CH_2-CH_2-NHR_{14}$.

On obtient un composé de formule (VIIIa) dans laquelle $X'_{1a}$ représente un groupe $-(CH_2)_p-NR_{14}C(=W_1)R_{16}$ dans lequel $W_1$ représente un atome de soufre à partir d'un composé de formule (VIIIa) correspondant, protégé sur l'azote de la pipéridine, et dans lequel $W_1$ représente un atome d'oxygène par réaction avec du pentasulfure de phosphore ou avec le réactif de Lawesson, le 2,4-bis(4-méthoxyphényl)-1,3-dithia-2,4-disphosphétane-2,4-disulfure, suivi de la déprotection de l'azote de la pipéridine.

Par action d'un chloroformiate de formule $ClCOOR_{17}$ sur un composé de formule (VIIIa) dans laquelle $X'_{1a}$ représente un groupe $-(CH_2)_m-NHR_{14}$, en présence d'une base telle que la triéthylamine, on prépare un composé de formule (VIIIa) dans laquelle $X'_{1a}$ représente un groupe $-(CH_2)_m-NR_{14}COOR_{17}$.

On peut également préparer un composé de formule (VIIIa) dans laquelle $X'_{1a}$ représente un groupe $-(CH_2)_m-NR_{14}COOR_{17}$ dans lequel m = 0 et $R_{14}$ représente l'hydrogène par action d'un composé $R_{17}OH$ avec un composé de formule (VIIIa) dans laquelle $X'_{1a}$ représente un groupe isocyanato, (-N = C = O).

On prépare un composé de formule (VIIIa) dans laquelle $X'_{1a}$ représente un groupe isocyanato à partir d'un

composé de formule (VIIIa) dans laquelle $X'_{1a}$ représente un carboxy selon la méthode décrite dans Organic Synthesis, 51, 48-52.

Par action d'un chlorure de sulfonyle $ClSO_2R_{18}$ sur un composé de formule (VIIIa) dans laquelle $X'_{1a}$ représente un groupe $-(CH_2)m-NHR_{14}$, en présence d'une base telle que la triéthylamine, on prépare un composé de formule (VIIIa) dans laquelle $X'_{1a}$ représente un groupe $-(CH_2)_m-NR_{14}SO_2R_{18}$.

De même par action d'un isocyanate de formule $R_{20}N=C=O$ dans lequelle $R_{20}$ représente un $(C_1-C_7)$alkyle, on prépare les composés de formule (VIIIa) dans laquelle $X'_{1a}$ représente un groupe $-(CH_2)_m-NR_{14}CONR_{19}R_{20}$ dans lequel $R_{19}$ représente un hydrogène et $R_{20}$ représente un $(C_1-C_7)$alkyle.

Par action d'un chlorure de carbamoyle de formule $ClCONR_{19}R_{20}$, on prépare les composés de formule (VIIIa) dans laquelle $X'_{1a}$ représente un groupe $-(CH_2)_m-NR_{14}CONR_{19}R_{20}$ dans lequel $R_{19}$ représente un $(C_1-C_7)$alkyle.

On peut également obtenir un composé de formule (VIIIa) dans laquelle $X'_{1a}$ représente un groupe $-(CH_2)_m-NR_{14}CONR_{19}R_{20}$ par action d'un composé $HNR_{19}R_{20}$ avec un composé de formule (VIIIa) dans laquelle $X'_{1a}$ représente un groupe $-(CH_2)_m-NR_{14}COOR_{17}$ dans lequel $R_{17}$ représente un phényle.

On peut aussi préparer un composé de formule (VIIIa) dans laquelle $X'_{1a}$ représente un groupe $-(CH_2)_m-NR_{14}CONR_{19}R_{20}$ dans lequel m = 0 et $R_{14}$ représente l'hydrogène par action d'un composé $NHR_{19}R_{20}$ avec un composé de formule (VIIIa) dans laquelle $X'_{1a}$ représente un groupe isocyanato.

Par réaction d'un composé de formule (VIIIa), protégé sur l'azote de la pipéridine, dans laquelle $X'_{1a}$ représente un groupe $-(CH_2)_m-NR_{14}CONR_{19}R_{20}$ avec du pentasulfure de phosphore ou avec le réactif de Lawesson, on prépare un composé de formule (VIIIa) dans laquelle $X'_{1a}$ représente un groupe $-(CH_2)_m-NR_{14}C(=W_1)NR_{19}R_{20}$ dans lequel $W_1$ est un atome de soufre.

Pour préparer un composé de formule (VIIIa) dans laquelle $X'_{1a}$ représente un groupe $-CONR_{19}R_{20}$, on fait réagir un composé de formule (VIIIa) dans laquelle $X'_{1a}$ représente un carboxy avec un composé de formule $HNR_{19}R_{20}$ selon les méthodes bien connues de l'homme de l'art.

De même, on prépare les composés de formule (VIIIa) dans laquelle $X'_{1a}$ représente un groupe $-CH_2-CONR_{19}R_{20}$, par réaction d'un composé de formule (VIIIa) dans laquelle $X'_{1a}$ représente un groupe $-CH_2-COOR_{21}$ dans lequel $R_{21}$ représente l'hydrogène avec un composé $HNR_{19}R_{20}$.

Selon les méthodes précédemment citées, on prépare un composé de formule (VIIIa) dans laquelle $X'_{1a}$ représente un groupe $(CH_2)_n-C(=W_1)NR_{19}R_{20}$ dans lequel $W_1$ représente un atome de soufre à partir d'un composé de formule (VIIIa) correspondante dans laquelle $W_1$ représente un atome d'oxygène.

On peut préparer un composé de formule (VIIIa) dans laquelle $X'_{1a}$ représente un carboxy par hydrolyse d'un composé de formule (VIIIa) dans laquelle $X'_{1a}$ représente un cyano selon les méthodes connues de l'homme de l'art.

On peut préparer un composé de formule (VIIIa) dans laquelle $X'_{1a}$ représente un carboxyméthyle selon la méthode décrite dans Chem. Ber., 1975, 108, 3475-3482.

On peut préparer un composé de formule (VIIIa) dans laquelle $X'_{1a}$ représente un $(C_1-C_7)$alcoxycarbonyle ou respectivement un $(C_1-C_7)$alcoxycarbonylméthyle à partir d'un composé de formule (VIIIa) dans laquelle $X'_{1a}$ représente un carboxy ou respectivement un carboxyméthyle, par réaction d'estérification selon les méthodes bien connues de l'homme de l'art.

On prépare un composé de formule (VIIIa) dans laquelle $X'_{1a}$ représente un groupe $-CO-NR_{22}-NR_{23}R_{24}$ par réaction d'une hydrazine $HNR_{22}-NR_{23}R_{24}$ avec un composé de formule (VIIIa) dans laquelle $X'_1$ représente un chloroformyle.

Pour préparer un composé de formule (VIIIa) dans laquelle $X'_{1a}$ représente un groupe :

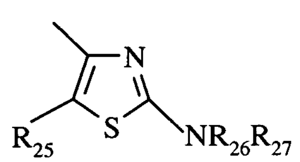

dans lequel $R_{26}$ et $R_{27}$ représentent chacun indépendamment un hydrogène ou un $(C_1-C_7)$alkyle, on fait réagir un composé de formule (VIIIa) dans laquelle $X'_{1a}$ représente un groupe

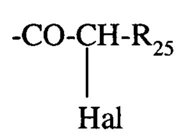

dans lequel Hal représente un atome d'halogène de préférence le brome, avec une thiourée dans laquelle un des groupes amino est libre ou substitué par un ou deux ($C_1$-$C_7$)alkyles.

On prépare un composé de formule (VIIIa) dans laquelle $X'_{1a}$ représente un groupe

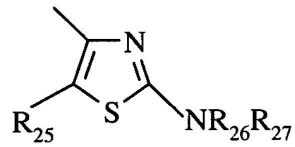

dans lequel $R_{27}$ représente un formyle ou respectivement un ($C_1$-$C_7$)alkylcarbonyle par réaction de l'acide formique dans l'anhydride acétique ou respectivement d'un chlorure d'acide ($C_1$-$C_7$)alkyl-COCl en présence d'une base telle que la triéthylamine, sur le composé de formule (VIIIa) ci-dessus protégé sur l'azote de la pipéridine et dans laquelle $R_{27}$ représente l'hydrogène. Après une étape de déprotection on obtient le composé attendu.

Le composé de formule (VIIIa) dans laquelle $X'_{1a}$ représente un groupe

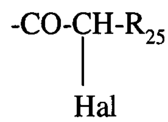

dans lequel Hal représente un atome de brome s'obtient par bromation selon les méthodes classiques d'un composé de formule (VIIIa) dans laquelle $X'_{1a}$ représente un groupe $-CO-CH_2-R_{25}$.

On peut préparer un composé de formule (VIIIa) dans laquelle $X'_{1a}$ représente un groupe

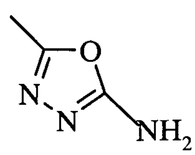

par réaction d'un composé de formule (VIIIa) protégé dans laquelle $X'_{1a}$ représente un groupe carbazoyle ($-CONH-NH_2$) avec le bromure de cyanogène selon la méthode décrite dans J. Org. Chem., 1961, 26, 88-95. Le composé de formule (Villa) dans laquelle $X'_{1a}$ représente un groupe carbazoyle s'obtient par réaction de l'hydrazine avec un composé de formule (Villa) dans laquelle $X'_{1a}$ représente un chloroformyle, lui-même obtenu par réaction du chlorure de thionyle avec un composé de formule (VIIIa) dans laquelle $X'_{1a}$ représente un carboxy.

Les énantiomères des composés selon l'invention, de formule :

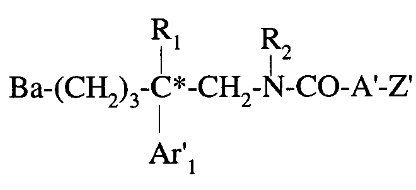
(Ia*) ;

dans laquelle :

- "*" signifie que l'atome de carbone ainsi marqué à la configuration absolue (+) ou (-) déterminée ;
- $Ar'_1$, A', Z' et Ba sont tels que définis pour les composés de formule (la) et $R_1$ et $R_2$ ensemble forment le groupe-$(CH_2)_3$- ;

ainsi que leurs sels avec des acides minéraux ou organiques,
sont des composés nouveaux qui font partie de l'invention.

La résolution des mélanges racémiques des composés de formule (la) permet d'isoler les énantiomères de

formule (Ia*). Il est cependant préférable d'effectuer le dédoublement des mélanges racémiques à partir d'un composé intermédiaires utile pour la préparation d'un composé de formule (Ia) tel que décrit dans les demandes de brevet : EP-A-0474561, EP-A-0512901, EP-A-0591040 et EP-A-0612716.

Les composés de formule (Ia) ci-dessus comprennent également ceux dans lesquels un ou plusieurs atomes d'hydrogène, de carbone ou d'iode ont été remplacés par leur isotope radioactif par exemple le tritium, le carbone-14 ou l'iode-125. De tels composés marqués sont utiles dans des travaux de recherche, de métabolisme ou de pharmacocinétique, dans des essais biochimiques en tant que ligands de récepteurs.

L'affinité des composés de formule (Ia) pour les récepteurs aux tachykinines a été évaluée in vitro par plusieurs essais biochimiques utilisant des radioligands :

1°) La liaison de [$^{125}$I] BH-SP (Substance P marquée à l'iode 125 à l'aide du réactif de Bolton-Hunter) aux récepteurs $NK_1$ du cortex de rat, de l'iléon de cobaye et des cellules lymphoblastiques humaines.

2°) La liaison de [$^{125}$I] His-$NK_A$ aux récepteurs $NK_2$ de vessie de rat ou la liaison de [$^{125}$I]NPγ aux récepteurs $NK_2$ de l'iléon de cobaye.

3°) La liaison de [$^{125}$I] His [MePhe$^7$] $NK_B$ aux récepteurs $NK_3$ du cortex cérébral de rat, du cortex cérébral de cobaye et du cortex cérébral de gerbille ainsi qu'aux récepteurs clonés $NK_3$ humains exprimés par des cellules CHO (Buell et al., FEBS Letters, 1992, 299, 90-95).

Les essais ont été effectués selon X. Emonds-Alt et al. (Eur. J. Pharmacol,. 1993, 250, 403-413).

Les composés selon l'invention inhibent fortement la liaison de [$^{125}$I]His[MePhe$^7$] $NK_B$ aux récepteurs $NK_3$ du cortex cérébral de cobaye et de gerbille ainsi qu'aux récepteurs clonés $NK_3$ humains : la constante d'inhibition Ki est généralement inférieure à $5.10^{-9}$M. Pour les mêmes composés on a constaté que la constante d'inhibition (Ki) pour les récepteurs $NK_3$ du cortex cérébral de rat est généralement supérieure à $10^{-8}$M et que la constante d'inhibition (Ki) pour le récepteur $NK_2$ du duodénum de rat et les récepteurs $NK_1$ du cortex de rat est généralement supérieure ou égale à $10^{-7}$ M.

Les composés selon la présente invention ont également été évalués in vivo sur deux modèles animaux.

Chez le gerbille, un comportement de rotation est induit par administration intrastriatale d'un agoniste spécifique du récepteur $NK_3$ : le senktide ; on a constaté qu'une application unilatérale de senktide dans le striatum de gerbille conduit à de fortes rotations contralatérales qui sont inhibées par les composés selon l'invention administrés soit par voie intrapéritonéale, soit par voie orale.

Ce résultat montre que les composés selon l'invention passent la barrière hématoméningée et qu'ils sont susceptibles de bloquer, au niveau du système nerveux central, l'action propre aux récepteurs $NK_3$. Ils pourront ainsi être utilisés pour le traitement de toute pathologie centrale $NK_B$ dépendante, telle que les maladies psychiatriques, ou de toute pathologie médiée au niveau central par le récepteur $NK_3$, telle que les maladies psychosomatiques.

Chez le cobaye, une injection de senktide par voie intraveineuse ou intracérébroventriculaire induit une hypertension qui est supprimée par l'administration par voie orale ou intraveineuse des composés selon l'invention.

Ce résultat montre que les composés selon l'invention agissent au niveau cardiovasculaire et qu'ils sont capables de bloquer l'action propre aux récepteurs $NK_3$ à ce niveau, notamment l'hypertension (Nakayama et al., Brain Res. 1992, 595 339-342, Takano and Kamiya, Asia Pacific. J. Pharmacol., 1991, 6, 341-346, Saigo et al., Neuroscience Letters, 1993, 159, 187-190).

Chez le cobaye, une inhalation, par exemple, de Substance P induit une hyperactivité bronchique à l'acétylcholine ainsi qu'une hypersensibilité à l'histamine par exemple de l'extravasation plasmatique. Un antagoniste $NK_3$ bloque ces deux processus caractéristiques des pathologies respiratoires comme l'asthme.

Dans ces tests, les composés selon l'invention sont actifs à des doses variant de 0,1 mg à 30 mg par kg par voie orale, intraveineuse ou intrapéritonéale.

Les composés de la présente invention sont généralement administrés en unité de dosage. Lesdites unités de dosage sont de préférence formulées dans des compositions pharmaceutiques dans lesquelles le principe actif est mélangé avec un excipient pharmaceutique.

Selon un autre de ses aspects, la présente invention concerne des compositions pharmaceutiques renfermant, en tant que principe actif, un composé de formule (Ia) ou un de ses sels pharmaceutiquement acceptables, ayant une affinité très forte pour le récepteur $NK_3$ humain, caractérisée par une constante d'inhibition Ki inférieure à $5.10^{-9}$M dans les études de fixation du ligand.

Les composés de formule (Ia) et leurs sels pharmaceutiquement acceptables peuvent être utilisés à des doses journalières de 0,01 à 100 mg par kilo de poids corporel du mammifère à traiter, préférentiellement à des doses journalières de 0,1 à 50 mg/kg. Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif.

Les maladies pour le traitement desquelles les composés et leurs sels pharmaceutiquement acceptables peuvent être utilisés, sont par exemple les maladies associées à un dysfonctionnement des systèmes dopaminergiques

telle que la schizophrénie, la maladie de Parkinson, les maladies associées à un dysfonctionnement des systèmes noradrénergiques et sérotoninergiques tels que l'anxiété, les troubles de la vigilance, de l'humeur, ainsi que les maladies épileptiques de toute forme et en particulier le Grand Mal, la démence, les maladies neurodégénératives, et les maladies périphériques dans lesquelles la participation du système nerveux central et/ou du système nerveux périphérique se fait par l'intermédiaire de la neurokinine B agissant comme neurotransmetteur ou neuromodulateur tels que la douleur, la migraine, l'inflammation aigue ou chronique, les troubles cardiovasculaires en particulier l'hypertension, l'insuffisance cardiaque, et les troubles du rythme, les troubles respiratoires (asthme, rhinite, toux, bronchites, allergies, hypersensibilité), les troubles du système gastrointestinal tels que ulcère oesophagique, colite, désordres liés au stress (stress-related disorders), syndrome du colon irritable (IBS), hypersécrétion acide (acidic secretion), emesis/nausée (consécutive à la chimiothérapie ou post opératoire, due au mal du transport ou aux troubles vestibulaires), les troubles du système urinaire (incontinence, vessie neurologique), les maladies du système immunitaire (arthrite rhumatoide), et plus généralement toute pathologie neurokinine B dépendante.

Dans les compositions pharmaceutiques de la présente invention pour l'administration par voie orale, sublinguale, inhalée, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange le principe actif principal avec un véhicule pharmaceutique tel que la silice, la gélatine, l'amidon, le lactose, le stéarate de magnésium, le talc, la gomme arabique ou analogues. On peut enrober les comprimés de saccharose, de divers polymères ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant tel qu'un glycol ou un ester de glycerol et en incorporant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec de liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions injectables qui contiennent des agents de dispersion et/ou des agents mouillants pharmacologiquement compatibles, par exemple le propylèneglycol ou le butylèneglycol.

Pour une administration par inhalation on utilise un aérosol contenant en outre, par exemple du trioléate de sorbitane ou de l'acide oléique ainsi que du trichlorofluorométhane, du dichlorofluorométhane, du dichlorotétrafluoroéthane ou tout autre gaz propulseur biologiquement compatible ; on peut également utiliser un système contenant le principe actif, seul ou associé à un excipient, sous forme de poudre.

Le principe actif peut être également présenté sous forme de complexe avec une cyclodextrine, par exemple α, β, γ-cyclodestrine, 2-hydroxypropyl-β-cyclodextrine, méthyl-β-cyclodextrine.

Le principe actif peut être formulé également sous forme de microcapsules, éventuellement avec un ou plusieurs supports ou additifs.

Dans chaque unité de dosage le principe actif de formule (Ia) est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,5 à 1000 mg de principe actif, de préférence de 2,5 à 250 mg devant être administrés une à quatre fois par jour.

Les compositions susdites peuvent également renfermer d'autres produits actifs utiles pour la thérapeutique souhaitée tels que, par exemple, des bronchodilatateurs, des antitussifs ou des antihistaminiques.

Grâce à leur très forte affinité pour le récepteur $NK_3$ humain et à leur grande sélectivité, les composés selon l'invention pourront être utilisés, sous forme radiomarquée comme réactifs de laboratoire.

Par exemple, ils permettent d'effectuer la caractérisation, l'identification et la localisation du récepteur $NK_3$ humain dans des coupes de tissus, ou du récepteur $NK_3$ chez l'animal entier par autoradiographie.

Les composés selon l'invention permettent également d'effectuer le tri ou screening des molécules en fonction de leur affinité pour le récepteur $NK_3$ humain. On opère alors par une réaction de déplacement du ligand radiomarqué,

objet de la présente invention de son récepteur $NK_3$ humain.

Dans les Préparations et dans les exemples on utilise les abréviations suivantes :

Me, OMe : méthyle, méthoxy
Et, OEt : éthyle, éthoxy
EtOH : éthanol
MeOH : méthanol
Ether : éther diéthylique
Ether iso : éther diisopropylique
DMF : diméthylformamide
DMSO : diméthylsulfoxyde
DCM : dichlorométhane
THF : tétrahydrofurane
AcOEt : acétate d'éthyle
$K_2CO_3$ : carbonate de potassium
$Na_2CO_3$ : carbonate de sodium
$KHCO_3$ : hydrogénocarbonate de potassium
$NaHCO_3$ : hydrogénocarbonate de sodium
NaCl : chlorure de sodium
$Na_2SO_4$ : sulfate de sodium
$MgSO_4$ : sulfate de magnésium
NaOH : soude
AcOH : acide acétique
$H_2SO_4$ : acide sulfurique
HCl : acide chlorhydrique
éther chlorhydrique : solution saturée d'acide chlorhydrique dans l'éther
BOP : benzotriazol-1-yloxytris(diméthylamino) phosphonium hexafluorophosphate
KCN : cyanure de potassium
DBU : 1,8-diazabicyclo[5.4.0]undec-7-ène
$NH_4Cl$ : chlorure d'ammonium
F : point de fusion
Eb : point d'ébullition
TA : température ambiante
silice H : gel de silice 60H commercialisé par Merck (DARMSTAD)
RMN : résonnance magnétique nucléaire
$\delta$ : déplacement chimique
s : singulet
se : singulet élargi
sd : singulet dédoublé
d : doublet
t : triplet
qd : quadruplet
sept : septuplet
mt : multiplet
m : massif

PREPARATION 1.1

Chlorhydrate de 4-(acryloyl-N-méthylamino)-4-phénylpipéridine.

A) 1-Benzyl-4-hydroxy-4-phénylpipéridine.

Ce composé est préparé par action du phényllithium sur la 1-benzylpipérid-4-one selon le procédé décrit dans EP-A-474561.

B) 4-Acétamido-1-benzyl-4-phénylpipéridine.

Ce composé est préparé par action de l'acétonitrile sur le composé obtenu à l'étape précédente selon le

procédé décrit dans EP-A-474561.

C) Dichlorhydrate de 4-amino-1-benzyl-4-phénylpipéridine.

On chauffe à reflux pendant 48 heures un mélange de 50 g du composé obtenu à l'étape précédente, 90 ml d'une solution d'HCl concentrée dans 210 ml d'eau. On concentre sous vide le mélange réactionnel, reprend le résidu dans un mélange EtOH/toluène et évapore sous vide les solvants. On dissout le résidu dans 100 ml de MeOH chaud, ajoute 500 ml d'acétone et laisse sous agitation en refroidissant au bain de glace. On essore les cristaux formés, les lave à l'acétone puis à l'éther et sèche. On obtient 48,9 g du produit attendu.

D) 1-Benzyl-4-(formylamino)-4-phénylpipéridine.

A une solution de 48,9 g du composé obtenu à l'étape précédente et 25 g de formiate de sodium dans 340 ml d'acide formique, on ajoute goutte à goutte 110 ml d'anhydride acétique puis laisse une nuit sous agitation à TA. On concentre sous vide le mélange réactionnel, reprend le résidu à l'eau, alcalinise par ajout d'une solution de NaOH concentrée, extrait au DCM, sèche la phase organique sur $MgSO_4$ et évapore sous vide le solvant. On obtient 38,8 g du produit attendu après cristallisation dans le mélange éther iso/pentane, F = 140°C.

E) 1-Benzyl-4-(méthylamino)-4-phénylpipéridine.

A une suspension de 12,5 g d'hydrure d'aluminium et de lithium dans 100 ml de THF, on ajoute lentement une solution de 38,8 g du composé obtenu à l'étape précédente dans 400 ml de THF et chauffe à reflux pendant 3 heures. Après refroidissement, on ajoute au mélange réactionnel une solution de 5 ml de NaOH concentrée dans 45 ml d'eau, filtre les sels minéraux et concentre sous vide le filtrat. On obtient 38 g du produit attendu.

F) 4-(Acryloyl-N-méthylamino)-1-benzyl-4-phénylpipéridine.

On refroidit à 0-5°C une solution de 1,5 g du composé obtenu à l'étape précédente, 1,5 ml de triéthylamine dans 40 ml de DCM, ajoute goutte à goutte 0,5 ml de chlorure d'acryloyle et laisse sous agitation en laissant remonter la température à TA. On verse le mélange réactionnel dans l'eau, après décantation lave la phase organique à l'eau, par une solution de NaOH 2N, sèche sur $MgSO_4$ et évapore sous vide le solvant. On obtient 1,3 g du produit attendu après cristallisation dans le mélange éther/pentane.

G) Chlorhydrate de 4-(acryloyl-N-méthylamino)-4-phénylpipéridine.

On refroidit à 0°C une solution de 1,3 g du composé obtenu à l'étape précédente dans 30 ml de 1,2-dichloroéthane, ajoute goutte à goutte 0,5 ml de chloroformiate de 1-chloroéthyle puis chauffe à reflux pendant 2 heures. On concentre sous vide le mélange réactionnel, reprend le résidu dans 15 ml de MeOH, chauffe à reflux pendant 30 minutes et concentre sous vide. On obtient 0,65 g du produit attendu après cristallisation dans l'AcOEt.

PREPARATION 1.2

*p*-Toluènesulfonate de 4-(2-aminothiazol-4-yl)-4-phénylpipéridine, monohydrate.

A) Bromhydrate de 4-(2-bromoacétyl)-4-phénylpipéridine.

A une suspension de 11,98 g de chlorhydrate de 4-acétyl-4-phénylpipéridine dans 200 ml de DCM on ajoute rapidement à TA 8 g de brome et laisse une nuit sous agitation à TA. On dilue le mélange réactionnel par ajout de 200 ml d'éther, essore le précipité formé et le lave à l'éther. On obtient 17,88 g du produit attendu après séchage sous vide.

B) *p*-Toluènesulfonate de 4-(2-aminothiazol-4-yl)-4-phénylpipéridine, monohydrate.

On chauffe à reflux pendant 3 heures un mélange de 7,26 g du composé obtenu à l'étape précédente, 1,52 g de thiourée dans 150 ml d'EtOH. On concentre sous vide le mélange réactionnel, reprend le résidu à l'eau, alcalinise à pH = 13 par ajout d'une solution de NaOH à 10 %, essore le précipité formé et le lave à l'eau puis à l'éther. On obtient 4,46 g du produit attendu sous forme de base libre après recristallisation dans l'EtOH. On dissout 1 g de la base dans l'acétone et ajoute 0,73 g d'acide *p*-toluènesulfonique monohydrate. On obtient 1,5 g du produit attendu cristallisé, F = 220-222°C.

PREPARATION 1.3

Chlorhydrate de 4-(2-hydroxyéthoxy)-4-phénylpipéridine.

A) 1-Benzyl-4-hydroxy-4-phénylpipéridine.

Ce composé est préparé par action du phényllithium sur la 1-benzylpipérid-4-one selon le procédé décrit dans EP-A-474561.

B) 4-(Benzoyloxy)-1-benzyl-4-phénylpipéridine.

On refroidit à 0-5°C une solution de 2,67 g du composé préparé à l'étape précédente, 2,5 ml de triéthylamine dans 30 ml de DCM, ajoute 1,22 ml de chlorure de benzoyle et laisse 1 heure sous agitation en laissant remonter la température à TA. On concentre sous vide le mélange réactionnel, extrait le résidu à l'AcOEt, lave la phase organique à l'eau, par une solution de NaOH 1N, sèche sur $MgSO_4$ et évapore sous vide le solvant. On obtient 2,4 g du produit attendu après cristallisation dans le pentane.

C) Chlorhydrate de 1-benzyl-4-(2-hydroxyéthoxy)-4-phénylpipéridine.

On chauffe à 60°C pendant 5 heures un mélange de 2,3 g du composé obtenu à l'étape précédente, 7 ml d'$H_2SO_4$ et 60 ml d'éthylène glycol. On verse le mélange réactionnel sur de la glace, alcalinise par ajout d'une solution de $NH_4OH$ concentrée, extrait au DCM, lave la phase organique à l'eau, sèche sur $MgSO_4$ et évapore sous vide le solvant. On chromatographie le résidu sur silice en éluant au DCM puis par le mélange DCM/MeOH (96/4 ; v/v). On dissout le produit obtenu dans du DCM, acidifie à pH = 1 par ajout d'éther chlorhydrique et essore le précipité formé. On obtient 1 g du produit attendu.

D) Chlorhydrate de 4-(2-hydroxyéthoxy)-4-phénylpipéridine.

On hydrogène à TA et à pression atmosphérique un mélange de 3,3 g du composé obtenu à l'étape précédente, 0,4 g de palladium sur charbon à 10 % dans 100 ml d'EtOH. On filtre le catalyseur sur Célite® et concentre sous vide le filtrat. On obtient 2,2 g du produit attendu, F = 168-172°C.

PREPARATION 1.4

Dibenzènesulfonate de 4-amino-4-phénylpipéridine.

On dissout 26,95 g du composé obtenu à l'étape C de la PREPARATION 1.3 dans 50 ml d'eau, alcalinise à pH = 12 par ajout d'une solution concentrée de NaOH, extrait au DCM, lave la phase organique par une solution saturée de NaCl, sèche sur $Na_2SO_4$ et évapore sous vide le solvant. On reprend l'huile obtenue dans 300 ml d'EtOH, ajoute 25 g d'acide benzènesulfonique et 2,2 g de palladium sur charbon à 5 % puis hydrogène à 40°C et à pression atmosphérique. On filtre le catalyseur sur Célite, lave au MeOH et concentre sous vide le filtrat. On reprend le résidu à l'acétone et essore le précipité formé. On obtient 29,7 g du produit attendu, F = 276-278°C.

PREPARATION 1.5

*p*-Toluènesulfonate de 4-(2-amino-1,3,4-oxadiazol-5-yl)-4-phénylpipéridine.

A) 1-(Benzyloxycarbonyl)-4-carboxy-4-phénylpipéridine.

On refroidit à 5°C un mélange de 37,7 g de *p*-toluènesulfonate de 4-carboxy-4-phénylpipéridine, 53,3 g d'une solution aqueuse à 30 % de NaOH et 250 ml d'eau. On ajoute rapidement à 5°C une solution de 18 g de chloroformiate de benzyle dans 60 ml d'acétone et laisse une nuit sous agitation en laissant remonter la température à TA. On lave deux fois le mélange réactionnel à l'éther et acidifie, après décantation, la phase aqueuse à pH = 1 par ajout d'HCl concentré puis d'HCl 2N. On essore le précipité formé, le sèche, le reprend à l'éther et l'essore à nouveau. On obtient 30,6 g du produit attendu, F = 142-144°C.

B) 1-(Benzyloxycarbonyl)-4-(chloroformyl)-4-phénylpipéridine.

On chauffe à reflux pendant 1 heure un mélange de 17,1 g du composé obtenu à l'étape précédente, 24 g de chlorure de thionyle dans 150 ml de 1,2-dichloroéthane. On concentre sous vide, reprend le résidu au chloroforme et évapore sous vide le solvant. On reprend le résidu dans un mélange éther/pentane et évapore à nouveau sous vide les solvants. On obtient 20 g du produit attendu sous forme de gomme que l'on utilise tel quel.

C) 1-(Benzyloxycarbonyl)-4-carbazoyl-4-phénylpipéridine.

On refroidit à -50°C une solution de 16 g d'hydrazine monohydrate dans 40 ml d'EtOH, ajoute goutte à goutte une solution de 11,44 g du composé obtenu à l'étape précédente dans 20 ml de 1,2-diméthoxyéthane et laisse sous agitation en laissant remonter la température à TA. On concentre sous vide, reprend le résidu à l'eau, extrait au DCM, lave la phase organique à l'eau, par une solution saturée de NaCl, sèche sur $MgSO_4$ et évapore sous vide le solvant. On reprend le résidu par un mélange EtOH/benzène et évapore sous vide les solvants. On obtient 11,2 g du produit attendu sous forme de gomme que l'on utilise tel quel.

D) 4-(2-Amino-1,3,4-oxadiazol-5-yl)-1-(benzyloxycarbonyl)-4-phénylpipéridine.

A une solution de 11,2 g du composé obtenu à l'étape précédente dans 60 ml d'EtOH, on ajoute à TA une solution de 3,39 g de bromure de cyanogène dans 10 ml d'EtOH et chauffe à reflux pendant 1 heure. On concentre le mélange réactionnel jusqu'à 50 ml d'EtOH, puis ajoute goutte à goutte de l'eau jusqu'à obtenir un volume de 400 ml de mélange réactionnel. On essore le produit cristallisé formé, le lave à l'eau puis au DCM, à l'AcOEt, et à l'éther. On obtient 8 g du produit attendu.

E) *p*-Toluènesulfonate de 4-(2-amino-1,3,4-oxadiazol-5-yl)-4-phénylpipéridine.

On hydrogène à 50°C et à pression atmosphérique un mélange de 7,85 g du composé obtenu à l'étape précédente, 3,95 g d'acide *p*-toluènesulfonique monohydrate, 0,8 g de palladium sur charbon à 10 %, 350 ml d'EtOH 95 et 10 ml d'eau. Après 3 heures, on filtre le catalyseur sur Célite® et concentre sous vide le filtrat. On reprend le résidu à l'acétone, essore le produit cristallisé formé et le lave à l'acétone puis à l'éther. On obtient 7,65 g du produit attendu, F = 183-185°C.

PREPARATION 2

Chlorhydrate de 3-(3,4-dichlorophényl)-3-(3-hydroxypropyl)pipéridine.

A) 4-Cyano-4-(3,4-dichlorophényl)heptanedioate de méthyle.

Dans un tricol, on dissout 37,2 g de 3,4-dichlorophénylacétonitrile et 34,43 g d'acrylate de méthyle dans 20 ml de dioxane ; on ajoute 1 ml de DBU, chauffe 2 heures à 60°C, évapore, dilue avec 400 ml d'acétate d'éthyle puis lave avec HCl dilué, une solution de NaCl, sèche sur $MgSO_4$ et évapore. Le produit attendu est cristallisé dans 100 ml d'acétate d'éthyle, et 100 ml d'éther avec 100 ml d'heptane. On obtient 47 g du produit.

B) 3-[5-(3,4-dichlorophényl)-2-oxopipérid-5-yl]propionate de méthyle.

On dissout 40 g du composé préparé à l'étape A dans 500 ml de 2-méthoxyéthanol, on ajoute 2 g de Nickel de Raney® et hydrogène à 40°C sous pression atmosphérique pendant 3 jours. On filtre, évapore, et obtient le produit attendu sous forme d'huile (39 g).

C) Acide 3-[5-(3,4-dichlorophényl)-2-oxopipérid-5-yl]propanoïque.

On dissout 17 g du composé préparé à l'étape précédente dans 250 ml de méthanol, ajoute 2,8 g de potasse et 10 ml d'eau puis on porte à reflux pendant 2 heures. On évapore à sec, reprend l'huile obtenue par 200 ml d'eau et lave par 100 ml d'acétate d'éthyle. La phase aqueuse est acidifiée par une solution d'HCl à 30 % puis on filtre et sèche le précipité formé. On recristallise dans le méthanol à chaud et obtient 18,3 g du composé attendu.

D) Chlorhydrate de 3-(3,4-dichlorophényl)-3-(3-hydroxypropyl)pipéridine.

On dissout 5 g du composé obtenu à l'étape précédente dans 20 ml de THF, ajoute 75 ml de borane (concentration 1 M dans le THF) et chauffe au reflux pendant 24 heures, sous azote. On ajoute 25 ml de méthanol, 50 ml d'HCl 4N et laisse sous agitation 30 minutes puis on ajoute de la soude à 40 % jusqu'à un pH supérieur à 10. On extrait 3 fois par 150 ml de DCM, sèche la phase organique sur $MgSO_4$ et évapore. Le résidu est mis en solution dans DCM avec une solution 4N d'HCl dans l'éther. Après évaporation, on obtient une mousse et le produit attendu (4,5 g) cristallise dans le mélange AcOEt/éther.

EXEMPLE 1

A) 1-Benzoyl-3-(3,4-dichlorophényl)-3-(3-hydroxypropyl)pipéridine.

On refroidit au bain de glace une solution de 16,22 g du composé obtenu à la PREPARATION 2, 18,2 g de triéthylamine dans 250 ml de DCM et ajoute, goutte à goutte, une solution de 14,06 g de chlorure de benzoyle dans 10 ml de DCM. On laisse 1 heure sous agitation en laissant remonter la température à TA. On élimine l'excès de chlorure de benzoyle par ajout de MeOH puis concentre sous vide le mélange réactionnel. On reprend le résidu au MeOH et évapore sous vide le solvant. On extrait le résidu à l'éther, lave à l'eau, par une solution d'HCl 2N, par une solution à 5 % de $NaHCO_3$, par une solution saturée de NaCl, sèche sur $MgSO_4$ et évapore sous vide. On dissout le 1-benzoyl-3-(3,4-dichlorophényl)-3-(3-benzoyloxypropyl) pipéridine ainsi obtenu intermédiairement dans 150 ml de MeOH, ajoute une solution de NaOH à 10 % , chauffe 1 heure à 50-60°C et concentre sous vide. On extrait le résidu à l'éther, lave à l'eau, par une solution d'HCl 2N, par une solution à 5 % de $NaHCO_3$, par une solution saturée de NaCl, sèche sur $MgSO_4$ et évapore sous vide le solvant. On obtient 18 g du produit attendu sous forme d'huile.

B) 1-Benzoyl-3-(3,4-dichlorophényl)-3-[3-méthanesulfonyloxy)propyl]pipéridine.

On refroidit au bain de glace une solution de 16,8 g du composé obtenu à l'étape précédente, 5,18 g de triéthylamine dans 100 ml de DCM et ajoute, goutte à goutte, une solution de 5,40 g de chlorure de méthanesulfonyle dans 10 ml de DCM puis laisse 30 minutes sous agitation en laissant remonter la température à TA. On concentre sous vide, extrait le résidu à l'AcOEt, lave à l'eau, par une solution d'HCl 2N, par une solution saturée de NaCl, sèche sur $MgSO_4$ et évapore sous vide le solvant. On obtient 19,6 g du produit attendu sous forme d'huile.
Spectre de RMN du proton à 200 MHz dans DMSO-$d_6$

1 à 2,35 ppm : m : 8H
3,15 ppm : s : 3H
3,2 à 4,6 ppm : m : 6H
6,8 à 7,8 ppm : m : 8H.

C) Chlorhydrate de 3-[3-[4-(acryloyl-N-méthylamino)-4-phénylpipérid-1-yl] propyl]-1-benzoyl-3-(3,4-dichlorophényl)pipéridine.

On chauffe à 80°C pendant 2 heures un mélange de 0,27 g de chlorhydrate de 4-(acryloyl-N-méthylamino)-4-phénylpipéridine, 0,45 g du composé obtenu à l'étape B ci-dessus, 0,3 g de $K_2CO_3$ dans 3 ml de DMF. On verse le mélange réactionnel dans l'eau, extrait à l'AcOEt, lave la phase organique à l'eau, par une solution saturée de NaCl, sèche sur $MgSO_4$ et évapore sous vide le solvant. On chromatographie le résidu sur silice en éluant par le gradient du mélange DCM/MeOH (99/1 ; v/v à 95/5 ; v/v). On dissout le produit obtenu dans du DCM, acidifie par ajout d'éther chlorhydrique et essore le précipité formé. On obtient 0,2 g du produit attendu, F = 128°C.

EXEMPLE 2

Dichlorhydrate de 3-[3-[4-(2-aminothiazol-4-yl)-4-phénylpipérid-1-yl] propyl]-1-benzoyl-3-(3,4-dichlorophényl)pipéridine, monohydrate.

On chauffe à reflux pendant 2 heures un mélange de 1,04 g de 4-(2-aminothiazol-4-yl)-4-phénylpipéridine (composé de la PREPARATION 1.2 sous forme de base libre), 1,88 g du composé obtenu à l'étape B de l'EXEMPLE 1, 1,1 g de $K_2CO_3$ dans 20 ml du mélange DMF/acétonitrile (50/50 ; v/v). On concentre sous vide le mélange réactionnel, reprend le résidu à l'eau, extrait à l'AcOEt, lave la phase organique à l'eau, sèche sur $MgSO_4$ et évapore sous vide le solvant. On chromatographie le résidu sur silice en éluant par le gradient du mélange DCM/MeOH (98/2 ; v/v à 95/5 ;

v/v). On reprend le produit obtenu dans de l'éther chlorhydrique et essore le précipité formé. On obtient 1,2 g du produit attendu après cristallisation dans l'AcOEt, F = 162-164°C.

EXEMPLE 3

Chlorhydrate de 1-benzoyl-3-(3,4-dichlorophényl)-3-[3-[4-(2-hydroxyéthoxy)-4-phénylpipérid-1-yl]propyl]pipéridine, monohydrate.

On chauffe à 60°C pendant 2 heures un mélange de 1 g de chlorhydrate de 4-(2-hydroxyéthoxy)-4-phénylpipéridine, 1,7 g du composé obtenu à l'étape B de l'EXEMPLE 1 et 0,65 g de $K_2CO_3$ dans 15 ml de DMF puis on laisse une nuit sous agitation à TA. On verse le mélange réactionnel dans l'eau, extrait au DCM, lave la phase organique à l'eau, sèche sur $Na_2SO_4$ et évapore sous vide le solvant. On chromatographie le résidu sur silice H en éluant au DCM puis par le mélange DCM/MeOH (96/4 ; v/v). On dissout le produit obtenu dans du DCM, acidifie par ajout d'éther chlorhydrique et essore le précipité formé. On obtient 1,2 g du produit attendu, F = 120-123°C.

EXEMPLE 4

Chlorhydrate de 3-[3-[4-(2-acétyloxyéthoxy)-4-phénylpipérid-1-yl]propyl]-1-benzoyl-3-(3,4-dichlorophényl)pipéridine, 1,5 hydrate.

On refroidit à 0-5°C une solution de 0,6 g du composé obtenu à l'EXEMPLE 3 et 0,5 ml de triéthylamine dans 25 ml de DCM, ajoute 0,085 ml de chlorure d'acétyle et on laisse 3 heures sous agitation en laissant remonter la température à TA. On concentre sous vide, reprend le résidu à l'eau, extrait à l'AcOEt, lave la phase organique à l'eau, sèche sur $Na_2SO_4$ et évapore sous vide le solvant. On chromatographie le résidu sur silice en éluant au DCM puis par le mélange DCM/MeOH (98/2 ; v/v). On dissout le produit obtenu dans du DCM, acidifie par ajout d'éther chlorhydrique et essore le précipité formé. On obtient 1,2 g du produit attendu, F = 105-107°C.

EXEMPLE 5

Chlorhydrate de 1-benzoyl-3-(3,4-dichlorophényl)-3-[3-[4-(2-furoylamino)-4-phénylpipérid-1-yl]propyl]pipéridine, hémihydrate.

A) 3-[3-(4-amino-4-phénylpipérid-1-yl)propyl]-1-benzoyl-3-(3,4-dichlorophényl) pipéridine.

On chauffe à 100°C pendant 5 heures un mélange de 6,81 g de dibenzènesulfonate de 4-amino-4-phénylpipéridine, 5,2 g du composé obtenu à l'étape B de l'EXEMPLE 1 et 6,1 g de $K_2CO_3$ dans 30 ml du mélange DMF/acétonitrile (50/50 ; v/v). On concentre sous vide, reprend le résidu à l'eau, extrait à l'AcOEt, lave la phase organique à l'eau, par une solution saturée de NaCl, sèche sur $MgSO_4$ et évapore sous vide le solvant. On chromatographie le résidu sur silice H en éluant par le mélange DCM/MeOH de (99/1 ; v/v) à (85/15 ; v/v). On obtient 3,6 g du produit attendu.

B) Chlorhydrate de 1-benzoyl-3-(3,4-dichlorophényl)-3-[3-[4-(2-furoylamino)-4-phénylpipérid-1-yl]propyl]pipéridine, hémihydrate.

On refroidit à 0-5°C une solution de 1,5 g du composé obtenu à l'étape précédente et 0,54 g de triéthylamine dans 10 ml de DCM, ajoute 0,35 g de chlorure de 2-furoyle et laisse 2 heures et 30 minutes sous agitation en laissant remonter la température à TA. On concentre sous vide, extrait le résidu au DCM, lave la phase organique à l'eau, par une solution à 5 % de $NaHCO_3$, à l'eau, par une solution saturée de NaCl, sèche sur $MgSO_4$ et évapore sous vide le solvant. On chromatographie le résidu sur silice H en éluant au DCM puis par le mélange DCM/MeOH (97/3 ; v/v). On dissout le produit obtenu dans l'AcOEt, fait barbotter un courant d'HCl gaz jusqu'à pH = 1 et ajoute de l'éther jusqu'à précipitation. On obtient 1,27 g du produit attendu après essorage et sèchage, F = 180-182°C.

EXEMPLE 6

Chlorhydrate de 1-benzoyl-3-(3,4-dichlorophényl)-3-[3-[4-(2-thénoylamino)-4-phénylpipérid-1-yl]propyl]pipéridine, monohydrate.

On prépare ce composé selon le mode opératoire décrit à l'étape B de l'EXEMPLE 5 à partir de 1,5 g du

composé obtenu à l'étape A de l'EXEMPLE 5, 0,55 g de triéthylamine et 0,4 g de chlorure de 2-thénoyle dans 10 ml de DCM. On chromatographie le résidu sur silice H en éluant au DCM puis par le mélange DCM/MeOH (95/5 ; v/v). On reprend le produit obtenu au DCM, acidifie à pH = 1 par ajout d'éther chlorhydrique et essore le précipité obtenu. On obtient 0,99 g du produit attendu, F = 198-200°C.

EXEMPLE 7

Dichlorhydrate de 1-benzoyl-3-(3,4-dichlorophényl)-3-[3-(4-pipéridinopipérid-1-yl)propyl]pipéridine, hémihydrate. (Ce composé n'est pas un composé de formule (Ia))

On chauffe à 100°C pendant 3 heures un mélange de 0,55 g de 4-pipéridinopipéridine, 1,3 g du composé obtenu à l'étape B de l'EXEMPLE 1, 1,14 g de $K_2CO_3$ dans 10 ml du mélange DMF/acétonitrile (50/50 ; v/v). On verse le mélange réactionnel dans l'eau, extrait à l'AcOEt, lave deux fois la phase organique à l'eau, par une solution saturée de NaCl, sèche sur $MgSO_4$ et évapore sous vide le solvant. On chromatographie le résidu sur silice H en éluant du DCM puis par le mélange DCM/MeOH (98/2 ; v/v). On dissout le produit obtenu dans l'AcOEt, fait barbotter un courant d'HCl gaz jusqu'à pH = 1 et ajoute de l'éther jusqu'à précipitation. On obtient 0,53 g du produit attendu après essorage et sèchage, F = 265°C (dèc).

En procédant selon les modes opératoires décrits dans les EXEMPLES précédents, à partir du composé obtenu à l'étape B de l'EXEMPLE 1 et à partir des pipéridines décrites dans les PREPARATIONS, on prépare les composés selon l'invention rassemblés dans le TABLEAU I ci-après.

TABLEAU I

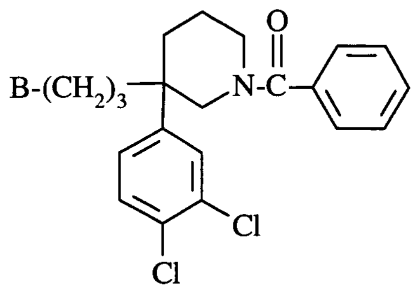

(I)

| Exemples | B- | Sel, solvate ; F°C ou RMN |
|---|---|---|
| 28<br><br>(a) | 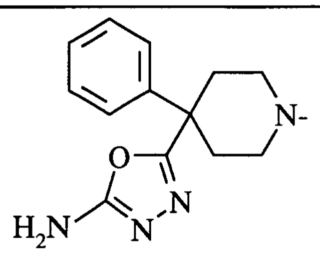 | 2 HCl, 2,5 $H_2O$ ;<br>202-204 |
| 29<br><br>(b) | 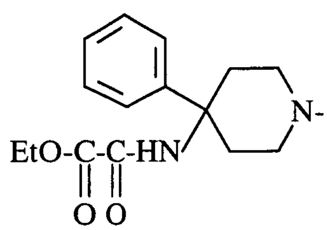 | HCl, $H_2O$ ;<br>160-162 |

(a) On prépare ce composé selon le mode opératoire décrit à l'EXEMPLE 7.

(b) On prépare ce composé selon le mode opératoire décrit à l'étape B de l'EXEMPLE 5, à partir du composé obtenu à l'étape A de l'EXEMPLE 5 et du chlorure d'éthyloxalyle.

## Revendications

1. Un composé de formule :

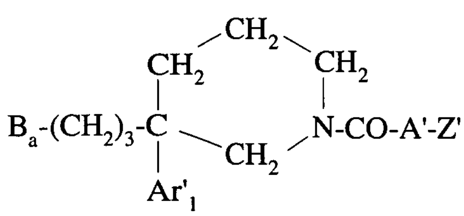

(Ia)

dans laquelle :

- $Ar'_1$ représente un phényle non substitué ou substitué une ou plusieurs fois par un substitutant choisi parmi :

un atome d'halogène, un hydroxy, un $(C_1-C_4)$alcoxy, un $(C_1-C_4)$alkyle, un trifluorométhyle, un méthylènedioxy, lesdits substituants étant identiques ou différents ;

- A' représente une liaison ou un groupe $-CH_2-$ ;
- Z' représente :

  . un phényle non substitué ou substitué une ou plusieurs fois par un substituant choisi parmi : un atome d'halogène ; un trifluorométhyle ; un cyano ; un hydroxy ; un nitro ; un amino non substitué ou substitué une ou deux fois par un $(C_1-C_4)$alkyle ; un benzylamino ; un carboxy ; un $(C_1-C_{10})$alkyle ; un $(C_3-C_8)$cycloalkyle non substitué ou substitué une ou plusieurs fois par un méthyle ; un $(C_1-C_{10})$alcoxy ; un $(C_3-C_8)$ cycloalkyloxy non substitué ou substitué une ou plusieurs fois par un méthyle ; un mercapto ; un $(C_1-C_{10})$ alkylthio ; un formyloxy ; un $(C_1-C_6)$alkylcarbonyloxy ; un formylamino ; un $(C_1-C_6)$ alkylcarbonylamino ; un benzoylamino ; un $(C_1-C_4)$alcoxycarbonyle ; un $(C_3-C_7)$ cycloalkyloxycarbonyle ; un carbamoyle non substitué ou substitué une ou deux fois par un $(C_1-C_4)$alkyle ; un uréido non substitué ou substitué une ou deux fois en position 3 par un $(C_1-C_4)$alkyle ou un $(C_3-C_7)$cycloalkyle ; un (pyrrolidin-1-yl)carbonylamino, lesdits substituants étant identiques ou différents ;

  . un naphtyle non substitué ou substitué une ou plusieurs fois par un halogène, un trifluorométhyle, un $(C_1-C_4)$alkyle, un hydroxy, un $(C_1-C_4)$alcoxy ;

  . un pyridyle; un thiényle; un indolyle ; un quinolyle ; un benzothiényle ; un imidazolyle ;

- $B_a$ représente un groupe $B_{1a}$ de formule :

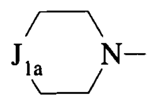

dans laquelle $J_{1a}$ représente un groupe

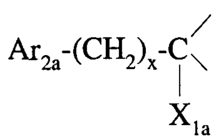

dans lequel :

- x est zéro ;
- $Ar_{2a}$ représente un phényle non substitué ou subsituté une ou plusieurs fois par un substituant choisi parmi : un atome d'halogène, un hydroxy, un $(C_1-C_4)$alcoxy, un $(C_1-C_4)$alkyle, un trifluorométhyle, un méthylènedioxy, lesdits substituants étant identiques ou différents ;
- $X_{1a}$ représente un groupe choisi parmi :

  . hydrogène ;

  . $(C_1-C_7)$alkyle ;

  . $-(CH_2)_m-OR_4$ dans lequel m est deux et $R_4$ représente un hydrogène ou un $(C_1-C_7)$alkyle ;

  . $-(CH_2)_m-OCOR_5$ dans lequel :

    m est deux et $R_5$ représente un hydrogène ; un $(C_1-C_7)$alkyle ; un $(C_3-C_7)$cycloalkyle non substitué ou substitué par un ou plusieurs méthyles ; un phényle ; un pyridyle ;ou

    m est zéro ou un et $R_5$ représente un $(C_3-C_7)$cycloalkyle non substitué ou substitué par un ou plusieurs méthyles ; un phényle ; un pyridyle ;

  . $-(CH_2)_m-OCONH(C_1-C_7)$alkyle dans lequel m est zéro ou deux ;

  . $-O-CH_2-CH_2-OR_6$ dans lequel $R_6$ représente un hydrogène ; un $(C_1-C_7)$alkyle ; un formyle ; un $(C_1-C_7)$ alkylcarbonyle ;

  . $-(CH_2)_n-SR_7$ dans lequel n est zéro ou un et $R_7$ représente un hydrogène ou un $(C_1-C_7)$alkyle ;

- $-CH_2-S(O)_j-(C_1-C_7)$alkyle dans lequel j est un ou deux ;
- $-NR_8R_9$ dans lequel $R_8$ et $R_9$ ensemble avec l'atome d'azote auquel ils sont liés constituent l'hétérocycle pipérazine non substitué ou substitué en position 4 par un $(C_1-C_4)$alkyle ;
- $-(CH_2)_p-NR_{10}R_{11}$ dans lequel p est deux et $R_{10}$ et $R_{11}$ représentent chacun indépendamment un hydrogène ou un $(C_1-C_7)$alkyle ; $R_{11}$ peut de plus représenter un $(C_3-C_7)$cycloalkylméthyle ou un benzyle ;
- $-NR_{12}COR_{13}$ dans lequel $R_{12}$ représente un hydrogène ou un $(C_1-C_7)$alkyle et $R_{13}$ représente un vinyle, un furyle, un thiényle, un pyrrolyle ou un imidazolyle ;
- $-NR_{14}COCOR_{15}$ dans lequel $R_{14}$ représente un hydrogène ou un $(C_1-C_7)$alkyle et $R_{15}$ représente un $(C_1-C_4)$alcoxy ;
- $-(CH_2)_p-NR_{14}C(=W_1)R_{16}$ dans lequel p est deux, $W_1$ représente un atome d'oxygène ou un atome de soufre, $R_{14}$ représente un hydrogène ou un $(C_1-C_7)$alkyle et $R_{16}$ représente un hydrogène, un $(C_1-C_7)$ alkyle ; un $(C_3-C_7)$cycloalkyle non substitué ou substitué par un ou plusieurs méthyles ; un phényle ; un benzyle ; un vinyle ; un pyridyle ; un furyle ; un thiényle ; un pyrrolyle ; un imidazolyle ; et p est un, $W_1$ représente un atome de soufre et $R_{14}$ et $R_{16}$ sont tels que l'on vient de les définir ou bien $W_1$ représente un atome d'oxygène, $R_{14}$ est tel que l'on vient de le définir et $R_{16}$ représente un vinyle ; un furyle, un thiényle, un pyrrolyle ou un imidazolyle ;
- $-(CH_2)_m-NR_{14}COOR_{17}$ dans lequel m est deux, $R_{14}$ représente un hydrogène ou un $(C_1-C_7)$alkyle et $R_{17}$ représente un $(C_1-C_7)$alkyle ou un phényle ;
- $-(CH_2)_m-NR_{14}SO_2R_{18}$ dans lequel m est deux, $R_{14}$ représente un hydrogène ou un $(C_1-C_7)$alkyle et $R_{18}$ représente un $(C_1-C_7)$alkyle ; un amino libre ou substitué par un ou deux $(C_1-C_7)$alkyles ; un phényle non substitué ou substitué une ou plusieurs fois par un substituant choisi parmi : un atome d'halogène, un $(C_1-C_7)$alkyle, un trifluorométhyle, un hydroxy, un $(C_1-C_7)$alcoxy, un carboxy, un $(C_1-C_7)$alkylcarbonyloxy, un cyano, un nitro, un amino libre ou substitué par un ou deux $(C_1-C_7)$alkyles, lesdits substituants étant identiques ou différents ;
- $-(CH_2)_m-NR_{14}C(=W_1)NR_{19}R_{20}$ dans lequel m est deux, $W_1$ représente un atome d'oxygène ou un atome de soufre, $R_{14}$ représente un hydrogène ou un $(C_1-C_7)$alkyle et $R_{19}$ et $R_{20}$ représentent chacun indépendamment un hydrogène ou un $(C_1-C_7)$alkyle ; $R_{20}$ peut de plus représenter un $(C_3-C_7)$cycloalkyle ; un $(C_3-C_7)$cycloalkylméthyle ; un hydroxy ; un $(C_1-C_4)$alcoxy ; un benzyle ; un phényle; un $(C_1-C_7)$alkyle substitué par un hydroxy, un $(C_1-C_3)$alcoxy, un phényle, un carboxy, un $(C_1-C_3)$alcoxycarbonyle ou un carbamoyle non substitué ou substitué par un ou deux $(C_1-C_7)$alkyles ; ou bien $R_{19}$ et $R_{20}$ ensemble avec l'atome d'azote auquel ils sont liés constituent un hétérocycle choisi parmi : l'azétidine, la pyrrolidine, la pipéridine, la morpholine, la thiomorpholine, la perhydroazépine ou la pipérazine non substituée ou substituée en position 4 par un $(C_1-C_4)$alkyle ; et m est zéro ou un, $W_1$ représente un atome de soufre et $R_{14}$, $R_{19}$ et $R_{20}$ sont tels que l'on vient de les définir ou bien $W_1$ représente un atome d'oxygène, $R_{14}$ et $R_{19}$ représentent chacun indépendamment un hydrogène ou un $(C_1-C_7)$alkyle et $R_{20}$ représente un $(C_1-C_7)$ alkyle substitué par un hydroxy, un $(C_1-C_3)$alcoxy, un phényle, un carboxy, un $(C_1-C_3)$alcoxycarbonyle ou un carbamoyle non substitué ou substitué par un ou deux $(C_1-C_7)$alkyles ; ou bien $R_{19}$ et $R_{20}$ ensemble avec l'atome d'azote auquel ils sont liés constituent l'hétérocycle pipérazine non substitué ou substitué en position 4 par un $(C_1-C_4)$alkyle ;
- $-(CH_2)_n-COOR_{21}$ dans lequel n est un et $R_{21}$ représente un hydrogène ou un $(C_1-C_7)$alkyle ; et n est zéro et $R_{21}$ représente un hydrogène ;
- $-(CH_2)_n-C(=W_1)NR_{19}R_{20}$ dans lequel n est un, $W_1$ représente un atome d'oxygène ou un atome de soufre et $R_{19}$ et $R_{20}$ représentent chacun indépendamment un hydrogène ou un $(C_1-C_7)$alkyle ; $R_{20}$ peut de plus représenter un $(C_3-C_7)$cycloalkyle ; un $(C_3-C_7)$cycloalkylméthyle ; un hydroxy ; un $(C_1-C_4)$alcoxy ; un benzyle ; un phényle ; un $(C_1-C_7)$alkyle substitué par un hydroxy, un $(C_1-C_3)$alcoxy, un phényle, un carboxy, un $(C_1-C_3)$alcoxycarbonyle ou un carbamoyle non substitué ou substitué par un ou deux $(C_1-C_7)$ alkyles ; ou bien $R_{19}$ et $R_{20}$ ensemble avec l'atome d'azote auquel ils sont liés constituent un hétérocycle choisi parmi : l'azétidine, la pyrrolidine, la pipéridine, la morpholine, la thiomorpholine, la perhydroazépine ou la pipérazine non substituée ou substituée en position 4 par un $(C_1-C_4)$alkyle ; et n est zéro; $W_1$ représente un atome de soufre et $R_{19}$ et $R_{20}$ sont tels que l'on vient de les définir ou bien $W_1$ représente un atome d'oxygène, $R_{19}$ représente un hydrogène ou un $(C_1-C_7)$alkyle et $R_{20}$ représente un $(C_1-C_7)$alkyle substitué par un hydroxy, un $(C_1-C_3)$alcoxy, un phényle, un carboxy, un $(C_1-C_3)$alcoxycarbonyle ou un carbamoyle non substitué ou substitué par un ou deux $(C_1-C_7)$alkyles ; ou bien $R_{19}$ et $R_{20}$ ensemble avec l'atome d'azote auquel ils sont liés constituent l'hétérocycle pipérazine non substitué ou substitué en position 4 par un $(C_1-C_4)$alkyle ;
- $-CO-NR_{22}NR_{23}R_{24}$ dans lequel $R_{22}$ représente un hydrogène ou un $(C_1-C_7)$alkyle et $R_{23}$ et $R_{24}$ représentent chacun indépendamment un hydrogène ou un $(C_1-C_7)$alkyle ;

.

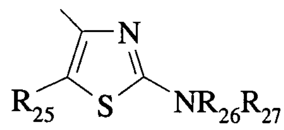

dans lequel $R_{25}$ représente un hydrogène ou un ($C_1$-$C_7$)alkyle, $R_{26}$ et $R_{27}$ représentent chacun indépendamment un hydrogène ou un ($C_1$-$C_7$)alkyle et $R_{27}$ peut de plus représenter un formyle ou un ($C_1$-$C_7$) alkylcarbonyle ;

.

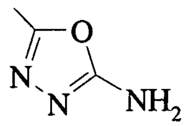 ;

ainsi que ses sels et solvates pharmaceutiquement acceptables.

**2.** Un composé selon la revendication 1 de formule :

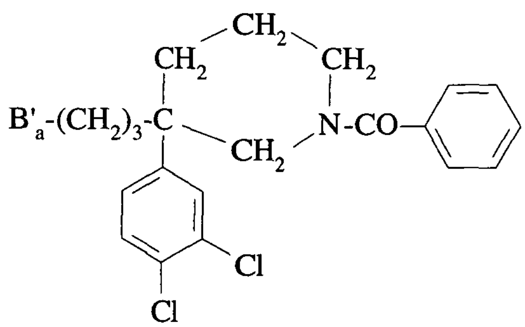

(I'a)

dans laquelle :

- $B'_a$ représente un groupe $B'_{1a}$ de formule :

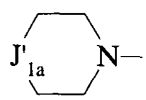

dans laquelle $J'_{1a}$ représente un groupe

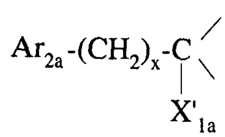

dans lequel :

- x est zéro ;
- $Ar_{2a}$ est tel que défini pour un composé de formule (Ia) dans la revendication 1 ;
- $X'_{1a}$ représente un groupe choisi parmi :

  . $-O-CH_2-CH_2-OR_6$ dans lequel $R_6$ représente un hydrogène ; un ($C_1$-$C_7$)alkyle ; un formyle ; un ($C_1$-$C_7$) alkylcarbonyle ;
  . $-NR_{12}COR_{13}$ dans lequel $R_{12}$ représente un hydrogène ou un ($C_1$-$C_7$)alkyle et $R_{13}$ représente un vinyle,

un furyle, un thiényle, un pyrrolyle ou un imidazolyle ;

. $-NR_{14}COCOR_{15}$ dans lequel $R_{14}$ représente un hydrogène ou un $(C_1-C_7)$alkyle et $R_{15}$ représente un $(C_1-C_4)$alcoxy ;

. $-(CH_2)_p-NR_{14}C(=W_1)R_{16}$ dans lequel p est un, $W_1$ représente un atome d'oxygène, $R_{14}$ représente un hydrogène ou un $(C_1-C_7)$alkyle et $R_{16}$ représente un vinyle ; un furyle ; un thiényle ; un pyrrolyle ou un imidazolyle ;

. $-(CH_2)_m-NR_{14}C(=W_1)NR_{19}R_{20}$ dans lequel m est zéro, $W_1$ représente un atome d'oxygène, $R_{14}$ représente un hydrogène ou un $(C_1-C_7)$alkyle, $R_{19}$ représente un hydrogène ou un $(C_1-C_7)$alkyle et $R_{20}$ représente un $(C_1-C_7)$alkyle substitué par un hydroxy, un $(C_1-C_3)$alcoxy, un phényle, un carboxy ; un $(C_1-C_3)$alcoxycarbonyle ou un carbamoyle non substitué ou substitué par un ou deux $(C_1-C_7)$alkyles ;

. $-CO-NR_{22}-NR_{23}R_{24}$ dans lequel $R_{22}$ représente un hydrogène ou un $(C_1-C_7)$alkyle et $R_{23}$ et $R_{24}$ représentent chacun indépendamment un hydrogène ou un $(C_1-C_7)$alkyle ;

. 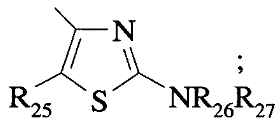 ;

dans lequel $R_{25}$ représente un hydrogène ou un $(C_1-C_7)$alkyle, $R_{26}$ et $R_{27}$ représentent chacun indépendamment un hydrogène ou un $(C_1-C_7)$alkyle et $R_{27}$ peut de plus représenter un formyle ou un $(C_1-C_7)$alkylcarbonyle ;

. 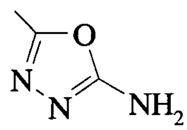 ;

et ses sels et solvates pharmaceutiquement acceptables.

**3.** Un composé selon l'une des revendications 1 ou 2, de formule :

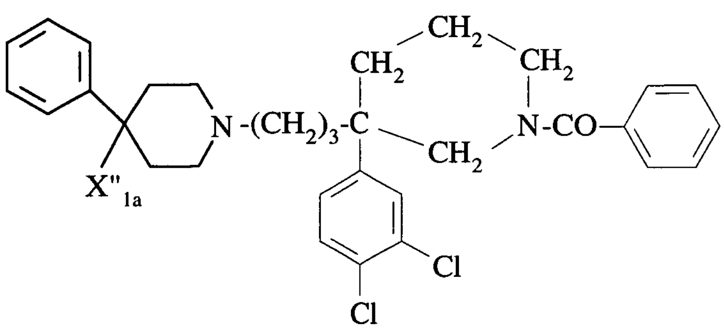

(I"a)

dans laquelle :

- X"$_{1a}$ représente un groupe choisi parmi :

. $-O-CH_2-CH_2-OR_6$ dans lequel $R_6$ représente un hydrogène ; un $(C_1-C_7)$alkyle ; un formyle ; un $(C_1-C_7)$alkylcarbonyle ; de préférence un hydrogène ou un acétyle ;

. $-NR_{12}COR_{13}$ dans lequel $R_{12}$ représente un hydrogène ou $(C_1-C_7)$alkyle, de préférence un hydrogène, et $R_{13}$ représente un vinyle, un furyle, un thiényle, un pyrrolyle ou un imidazolyle, de préférence un furyle ou un thiényle ;

. $-NR_{14}COCOR_{15}$ dans lequel $R_{14}$ représente un hydrogène ou un $(C_1-C_7)$alkyle, de préférence un hydrogène, et $R_{15}$ représente un $(C_1-C_4)$alcoxy, de préférence un éthoxy ;

- 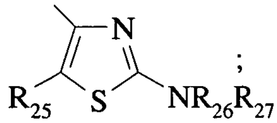 ;

dans lequel $R_{25}$ représente un hydrogène ou un ($C_1$-$C_7$)alkyle, de préférence un hydrogène, et $R_{26}$ et $R_{27}$ représentent chacun indépendamment un hydrogène ou un ($C_1$-$C_7$)alkyle et $R_{27}$ peut de plus représenter un formyle ou un ($C_1$-$C_7$)alkylcarbonyle, de préférence $R_{26}$ et $R_{27}$ représentent un hydrogène ;

- 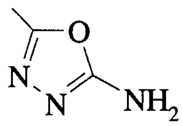 ;

et ses sels et solvates, pharmaceutiquement acceptables.

**4.** Un composé selon l'une quelconque des revendications 1, 2 ou 3 choisi parmi :

la 3-[3-[4-(acryloyl-N-méthylamino)-4-phénylpipérid-1-yl]propyl]-1-benzoyl-3-(3,4-dichlorophényl)pipéridine ;
la 3-[3-[4-(2-aminothiazol-4-yl)-4-phénylpipérid-1-yl]propyl]-1-benzoyl-3-(3,4-dichlorophényl)pipéridine ;
la 1-benzoyl-3-(3,4-dichlorophényl)-3-[3-[4-(2-hydroxyéthoxy)-4-phénylpipérid-1-yl]propyl]pipéridine ;
la 3-[3-[4-(2-acétyloxyéthoxy)-4-phénylpipérid-1-yl]propyl]-1-benzoyl-3-(3,4-dichlorophényl)pipéridine ;
la 1-benzoyl-3-(3,4-dichlorophényl)-3-[3-[4-(2-furoylamino)-4-phénylpipérid-1-yl]propyl]pipéridine ;
la 1-benzoyl-3-(3,4-dichlorophényl)-3-[3-[4-(2-thénoylamino)-4-phénylpipérid-1-yl]propyl]pipéridine ;
la 3-[3-[4-(2-amino-1,3,4-oxadiazol-5-yl)-4-phénylpipérid-1-yl]propyl]-1-benzoyl-3-(3,4-dichlorophényl)pipéridine ;
la 1-benzoyl-3-(3,4-dichlorophényl)-3-[3-[4-(éthoxalylamino)-4-phénylpipérid-1-yl]propyl]pipéridine ;

sous forme de racémates ou de l'un de leurs énantiomères (+) ou (-),
et leurs sels avec des acides minéraux ou organiques.

**5.** Solvates des composés selon l'une quelconque des revendications 1 à 4 et des sels de ceux-ci.

**6.** Procédé pour la préparation d'un composé de formule (Ia) selon la revendication 1 et de ses sels, **caractérisé en ce que** :

1) on traite un composé de formule :

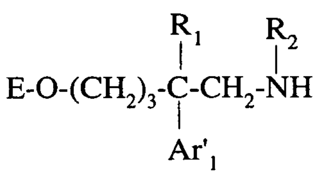 (II)

dans laquelle $Ar'_1$ est tel que défini pour un composé de formule (Ia) dans la revendication 1, E représente l'hydrogène ou un groupe O-protecteur et $R_1$ et $R_2$ ensemble forment le groupe $-(CH_2)_3-$
pour obtenir un composé de formule :

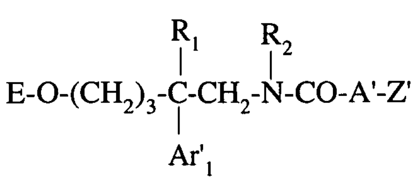 (IV)

2) on élimine éventuellement le groupe O-protecteur du composé de formule (IV), par action d'un acide ou d'une base, pour obtenir l'alcool de formule :

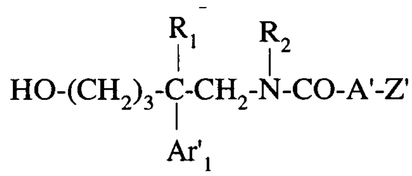 (V)

3) on traite l'alcool (V) avec un composé de formule :

$G-SO_2-Cl$ (Vi)

dans laquelle G représente un groupe méthyle, phényle, tolyle ou trifluorométhyle,
pour obtenir un composé de formule :

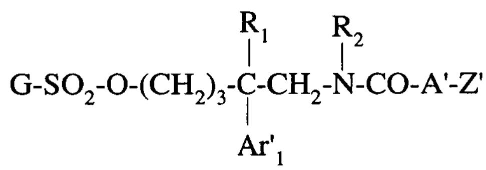 (VII)

4) on fait réagir le composé (VII)
avec une amine secondaire cyclique de formule :

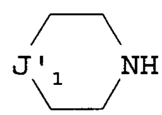 (VIIIa)

dans laquelle $J'_1$ représente :
un groupe

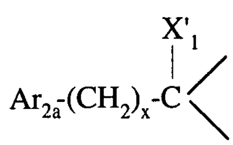

dans lequel $Ar_{2a}$ et x sont tels que définis pour (Ia) et $X'_1$ représente soit $X_{1a}$ tel que défini pour (Ia), soit un précurseur de $X_{1a}$, étant entendu que lorsque $X_{1a}$ contient un groupe hydroxyle ou un groupe aminé, ces groupes peuvent être protégés ;
5) et, après déprotection éventuelle des groupes hydroxyles ou des groupes aminés, ou transformation éventuelle de $X'_{1a}$ en $X_{1a}$, on transforme éventuellement le produit ainsi obtenu en l'un de ses sels avec un acide minéral ou organique.

7. Procédé pour la préparation d'un composé de formule (Ia) selon la revendication 1 et de ses sels, **caractérisé en ce que** :

1') on protège l'atome d'azote du composé de formule (II) tel que défini dans la revendication 5, pour obtenir un composé de formule :

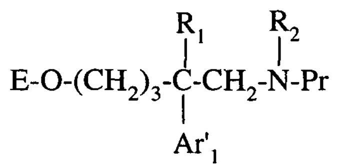 (XVII)

dans laquelle Ar'$_1$ est tel que défini pour un composé de formule (Ia) dans la revendication 1, E représente l'hydrogène ou un groupe O-protecteur, Pr représente un groupe N-protecteur, tel que le groupe trityle, *tert*-butoxycarbonyle ou benzyloxycarbonyle et $R_1$ et $R_2$ forment ensemble un groupe —$(CH_2)_3$- ,
2') on élimine éventuellement le groupe O-protecteur du composé de formule (XVII), par action d'un acide ou d'une base, pour obtenir l'acool de formule :

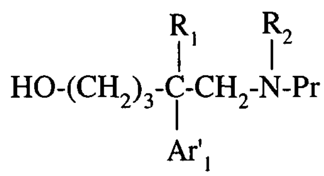 (XVIII)

3') on traite l'alcool (XVIII) avec un composé de formule (VI) tel que défini dans la revendication 25, pour obtenir un composé de formule :

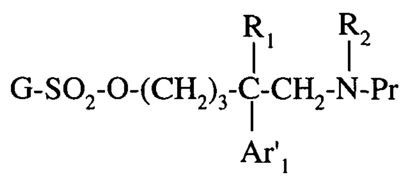 (XIX)

4') on fait réagir le composé (XIX) avec un composé de formule (VIIIa) tel que défini dans la revendication 6, pour obtenir un composé de formule :

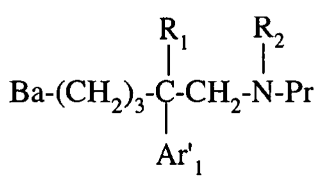 (XX)

dans laquelle Ba est tel que défini pour un composé de formule (Ia) dans la revendication 1 étant entendu que lorsque Ba contient un groupe hydroxyde ou un groupe aminé, ces groupes peuvent être protégés ;
5') on élimine sélectivement le groupe protecteur Pr du composé de formule (XX), pour obtenir le composé de formule :

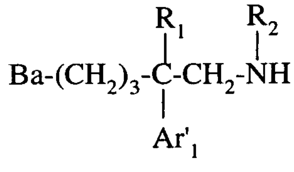 (XXI)

6') on traite le composé de formule (XXI) avec un composé de formule (IIIa), tel que défini dans la revendication 6 ;
7') et, après déprotection éventuelle des groupes hydroxyles ou des groupes aminés, on transforme éventuellement le produit ainsi obtenu en l'un de ses sels avec un acide minéral ou organique.

**8.** Un énantiomère d'un composé selon la revendication 1 de formule :

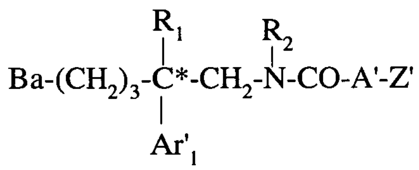

(Ia*)

dans laquelle :

- "*" signifie que l'atome de carbone ainsi marqué à la configuration absolue (+) ou (-) déterminée ;
- Ar'$_1$, A', Z' et Ba sont tels que définis pour les composés de formule (Ia) dans la revendication 1 et $R_1$ et $R_2$ ensemble forment le groupe-$(CH_2)_3$- ; ainsi que ses sels avec des acides minéraux ou organiques et ses solvates.

**9.** Composition pharmaceutique comprenant en tant que principe actif un composé selon l'une quelconque des revendications 1 à 4 ou 8 ou un de ses sels et solvates pharmaceutiquement acceptables.

**10.** Composition pharmaceutique selon la revendication 9, sous forme d'unité de dosage, dans laquelle le principe actif est mélangé à au moins un excipient pharmaceutique.

**11.** Composition pharmaceutique selon la revendication 10, contenant 0,5 à 1000 mg de principe actif.

**12.** Composition pharmaceutique selon la revendication 11, contenant 2,5 à 250 mg de principe actif.

Office européen des brevets

# RAPPORT DE RECHERCHE EUROPEENNE

Numéro de la demande
EP 02 01 0824

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X,P, D | EP 0 673 928 A (SANOFI) 27 septembre 1995 (1995-09-27) * exemple 27 * --- | 1,9 | C07D401/14 C07D401/06 C07D417/14 A61K31/445 C07D211/58 C07D211/52 //(C07D417/14, 277:00,211:00, 211:00), (C07D417/14, 271:00,211:00, 211:00) |
| X,D | EP 0 512 901 A (ELF SANOFI) 11 novembre 1992 (1992-11-11) * exemple 26 * --- | 1,9 | |
| Y,D | EP 0 428 434 A (SANOFI S.A.) 22 mai 1991 (1991-05-22) * revendications; exemples 104,106,146 * --- | 1-12 | |
| Y,D | WO 94 26735 A (MERRELL DOW PHARMACEUTICALS INC.) 24 novembre 1994 (1994-11-24) * revendications; exemples 61,62 * --- | 1-12 | |
| Y | WO 93 18002 A (ELF SANOFI) 16 septembre 1993 (1993-09-16) * tableau A, dernier composé, page 27 * --- | 1-12 | |
| A,D | EP 0 625 509 A (ZENECA LIMITED) 23 novembre 1994 (1994-11-23) * le document en entier * --- | 1-12 | **DOMAINES TECHNIQUES RECHERCHES (Int.Cl.7)** |
| A,D | EP 0 474 561 A (SANOFI) 11 mars 1992 (1992-03-11) * exemple 22 * --- | 1-12 | C07D A61K |
| A,D | EP 0 515 240 A (SANOFI) 25 novembre 1992 (1992-11-25) * revendications * --- | 1-12 | |
| A,D | LIFE SCIENCES, vol. 56, no. 1, 1995, pages PL27-32, XP002005529 * le document en entier * --- -/-- | 1-12 | |

Le présent rapport a été établi pour toutes les revendications

| Lieu de la recherche | Date d'achèvement de la recherche | Examinateur |
|---|---|---|
| BERLIN | 24 juillet 2002 | Frelon, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

Office européen des brevets

# RAPPORT DE RECHERCHE EUROPEENNE

Numéro de la demande
EP 02 01 0824

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | CHEMICAL ABSTRACTS, vol. 123, no. 11, 11 septembre 1995 (1995-09-11) Columbus, Ohio, US; abstract no. 132661j, XP002005530 * abrégé * & F. OURY-DONAT ET AL.: J. PHARMACOL. EXP. THER., vol. 274, no. 1, 1995, pages 148-154, ----- | 1-12 | |

DOMAINES TECHNIQUES RECHERCHES (Int.Cl.7)

Le présent rapport a été établi pour toutes les revendications

| Lieu de la recherche | Date d'achèvement de la recherche | Examinateur |
|---|---|---|
| BERLIN | 24 juillet 2002 | Frelon, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO. EP 02 01 0824

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

24-07-2002

| Document brevet cité au rapport de recherche | Date de publication | Membre(s) de la famille de brevet(s) | Date de publication |
|---|---|---|---|
| EP 673928 A | 27-09-1995 | FR 2717477 A1 | 22-09-1995 |
| | | FR 2717478 A1 | 22-09-1995 |
| | | FR 2719311 A1 | 03-11-1995 |
| | | AT 204863 T | 15-09-2001 |
| | | AU 693845 B2 | 09-07-1998 |
| | | AU 1490995 A | 28-09-1995 |
| | | CA 2145000 A1 | 19-09-1995 |
| | | CN 1128756 A ,B | 14-08-1996 |
| | | DE 69522355 D1 | 04-10-2001 |
| | | DE 69522355 T2 | 23-05-2002 |
| | | DK 673928 T3 | 12-11-2001 |
| | | EP 0673928 A1 | 27-09-1995 |
| | | ES 2164746 T3 | 01-03-2002 |
| | | FI 951265 A | 19-09-1995 |
| | | HU 72065 A2 | 28-03-1996 |
| | | IL 113026 A | 20-06-1999 |
| | | JP 2922816 B2 | 26-07-1999 |
| | | JP 8048669 A | 20-02-1996 |
| | | KR 238343 B1 | 02-03-2000 |
| | | NO 951044 A | 19-09-1995 |
| | | NO 975089 A | 19-09-1995 |
| | | NZ 270727 A | 26-05-1997 |
| | | PL 307723 A1 | 02-10-1995 |
| | | PT 673928 T | 28-02-2002 |
| | | RU 2143425 C1 | 27-12-1999 |
| | | TW 380138 B | 21-01-2000 |
| | | US 6124316 A | 26-09-2000 |
| | | US 5741910 A | 21-04-1998 |
| | | US 5942523 A | 24-08-1999 |
| | | ZA 9502228 A | 21-12-1995 |
| EP 512901 A | 11-11-1992 | FR 2676055 A1 | 06-11-1992 |
| | | AT 181550 T | 15-07-1999 |
| | | AU 652046 B2 | 11-08-1994 |
| | | AU 1591692 A | 05-11-1992 |
| | | BR 9201656 A | 15-12-1992 |
| | | CA 2067877 A1 | 04-11-1992 |
| | | CS 9201329 A3 | 18-11-1992 |
| | | DE 69229460 D1 | 29-07-1999 |
| | | DE 69229460 T2 | 20-01-2000 |
| | | DK 512901 T3 | 06-12-1999 |
| | | EP 0512901 A1 | 11-11-1992 |
| | | ES 2137176 T3 | 16-12-1999 |
| | | FI 921951 A | 04-11-1992 |
| | | FI 951242 A | 16-03-1995 |
| | | FI 951243 A | 16-03-1995 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 02 01 0824

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

24-07-2002

| Document brevet cité au rapport de recherche | Date de publication | Membre(s) de la famille de brevet(s) | Date de publication |
|---|---|---|---|
| EP 512901 A | | GR 3030755 T3 | 30-11-1999 |
| | | HK 1005138 A1 | 12-05-2000 |
| | | HU 61539 A2 | 28-01-1993 |
| | | IE 921364 A1 | 04-11-1992 |
| | | IL 101760 A | 18-02-1997 |
| | | IL 117921 A | 18-02-1997 |
| | | JP 3242980 B2 | 25-12-2001 |
| | | JP 5186425 A | 27-07-1993 |
| | | KR 258373 B1 | 01-07-2000 |
| | | MX 9202027 A1 | 01-01-1993 |
| | | NO 921734 A ,B, | 04-11-1992 |
| | | NZ 242586 A | 26-10-1995 |
| | | RU 2083574 C1 | 10-07-1997 |
| | | US 5770735 A | 23-06-1998 |
| | | US 5625060 A | 29-04-1997 |
| | | US 5340822 A | 23-08-1994 |
| | | ZA 9203178 A | 27-01-1993 |
| EP 428434 A | 22-05-1991 | FR 2654100 A1 | 10-05-1991 |
| | | FR 2663329 A1 | 20-12-1991 |
| | | AU 668018 B2 | 18-04-1996 |
| | | AU 5924594 A | 02-06-1994 |
| | | AU 649973 B2 | 09-06-1994 |
| | | AU 6583890 A | 23-05-1991 |
| | | CA 2029275 A1 | 07-05-1991 |
| | | EP 0428434 A2 | 22-05-1991 |
| | | FI 97540 B | 30-09-1996 |
| | | FI 952956 A | 15-06-1995 |
| | | FI 952957 A | 15-06-1995 |
| | | FI 980227 A | 02-02-1998 |
| | | HU 56543 A2 | 30-09-1991 |
| | | IE 903957 A1 | 08-05-1991 |
| | | IL 96241 A | 31-03-1996 |
| | | IL 111292 A | 31-03-1996 |
| | | JP 3206086 A | 09-09-1991 |
| | | LV 10713 A | 20-06-1995 |
| | | LV 10713 B | 20-10-1995 |
| | | MX 9203638 A1 | 01-09-1992 |
| | | NO 904802 A ,B, | 07-05-1991 |
| | | NO 950239 A ,B, | 07-05-1991 |
| | | NO 950240 A ,B, | 07-05-1991 |
| | | NZ 235985 A | 26-10-1993 |
| | | NZ 245411 A | 26-10-1993 |
| | | PL 166565 B1 | 30-06-1995 |
| | | PL 166582 B1 | 30-06-1995 |
| | | PT 95790 A | 13-09-1991 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 02 01 0824

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

24-07-2002

| Document brevet cité au rapport de recherche | Date de publication | Membre(s) de la famille de brevet(s) | Date de publication |
|---|---|---|---|
| EP 428434 A | | RU 2084453 C1 | 20-07-1997 |
| | | US 5686609 A | 11-11-1997 |
| | | US 5618938 A | 08-04-1997 |
| | | US 5317020 A | 31-05-1994 |
| | | ZA 9008881 A | 28-08-1991 |
| | | PL 165758 B1 | 28-02-1995 |
| | | PL 165854 B1 | 28-02-1995 |
| | | RU 2114828 C1 | 10-07-1998 |
| WO 9426735 A | 24-11-1994 | AT 158580 T | 15-10-1997 |
| | | AU 678023 B2 | 15-05-1997 |
| | | AU 6942694 A | 12-12-1994 |
| | | CA 2160462 A1 | 24-11-1994 |
| | | CN 1124961 A ,B | 19-06-1996 |
| | | DE 69405862 D1 | 30-10-1997 |
| | | DE 69405862 T2 | 15-01-1998 |
| | | DK 696280 T3 | 12-01-1998 |
| | | EP 0696280 A1 | 14-02-1996 |
| | | ES 2110761 T3 | 16-02-1998 |
| | | FI 955258 A | 30-11-1995 |
| | | GR 3025475 T3 | 27-02-1998 |
| | | HU 74085 A2 | 28-11-1996 |
| | | IL 109496 A | 26-07-2000 |
| | | JP 9500361 T | 14-01-1997 |
| | | MX 9403386 A1 | 31-01-1995 |
| | | NO 954400 A | 08-01-1996 |
| | | NZ 267209 A | 22-09-1997 |
| | | WO 9426735 A1 | 24-11-1994 |
| | | US 5635510 A | 03-06-1997 |
| | | US 5648366 A | 15-07-1997 |
| | | US 5661160 A | 26-08-1997 |
| | | US 5861416 A | 19-01-1999 |
| | | US 5824690 A | 20-10-1998 |
| | | ZA 9403091 A | 12-01-1995 |
| WO 9318002 A | 16-09-1993 | FR 2688218 A1 | 10-09-1993 |
| | | FR 2688219 A1 | 10-09-1993 |
| | | AT 193015 T | 15-06-2000 |
| | | CA 2090785 A1 | 04-09-1993 |
| | | DE 69328643 D1 | 21-06-2000 |
| | | DE 69328643 T2 | 28-12-2000 |
| | | EP 0559538 A1 | 08-09-1993 |
| | | WO 9318002 A1 | 16-09-1993 |
| | | HU 70167 A2 | 28-09-1995 |
| | | JP 6507425 T | 25-08-1994 |
| | | MX 9301158 A1 | 01-09-1993 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 02 01 0824

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

24-07-2002

| Document brevet cité au rapport de recherche | | Date de publication | Membre(s) de la famille de brevet(s) | | | Date de publication |
|---|---|---|---|---|---|---|
| WO 9318002 | A | | US | 5674881 | A | 07-10-1997 |
| | | | US | 5773620 | A | 30-06-1998 |
| EP 625509 | A | 23-11-1994 | CA | 2123636 | A1 | 18-11-1994 |
| | | | DE | 69404549 | D1 | 04-09-1997 |
| | | | DE | 69404549 | T2 | 18-12-1997 |
| | | | EP | 0625509 | A1 | 23-11-1994 |
| | | | JP | 6340624 | A | 13-12-1994 |
| | | | US | 5521199 | A | 28-05-1996 |
| EP 474561 | A | 11-03-1992 | FR | 2666335 | A1 | 06-03-1992 |
| | | | FR | 2678267 | A1 | 31-12-1992 |
| | | | AT | 174332 | T | 15-12-1998 |
| | | | AU | 657272 | B2 | 09-03-1995 |
| | | | AU | 8354291 | A | 12-03-1992 |
| | | | BR | 9103802 | A | 19-05-1992 |
| | | | CA | 2050639 | A1 | 06-03-1992 |
| | | | CS | 9102724 | A3 | 18-03-1992 |
| | | | DE | 69130597 | D1 | 21-01-1999 |
| | | | DE | 69130597 | T2 | 20-05-1999 |
| | | | DK | 474561 | T3 | 16-08-1999 |
| | | | EP | 0474561 | A1 | 11-03-1992 |
| | | | ES | 2127722 | T3 | 01-05-1999 |
| | | | FI | 914174 | A ,B, | 06-03-1992 |
| | | | GR | 3029435 | T3 | 28-05-1999 |
| | | | HK | 1005290 | A1 | 18-08-2000 |
| | | | HU | 59098 | A2 | 28-04-1992 |
| | | | HU | 9500521 | A3 | 30-10-1995 |
| | | | IE | 913082 | A1 | 11-03-1992 |
| | | | IL | 99320 | A | 31-07-1995 |
| | | | JP | 2620435 | B2 | 11-06-1997 |
| | | | JP | 4261155 | A | 17-09-1992 |
| | | | LT | 585 | A ,B | 27-12-1994 |
| | | | LV | 10606 | A | 20-04-1995 |
| | | | LV | 10606 | B | 20-04-1996 |
| | | | NO | 913469 | A ,B, | 06-03-1992 |
| | | | NZ | 239661 | A | 27-06-1994 |
| | | | PL | 167994 | B1 | 30-12-1995 |
| | | | PT | 98849 | A ,B | 31-07-1992 |
| | | | SG | 47703 | A1 | 17-04-1998 |
| | | | RU | 2070196 | C1 | 10-12-1996 |
| | | | US | 5350852 | A | 27-09-1994 |
| | | | US | 5236921 | A | 17-08-1993 |
| | | | ZA | 9107017 | A | 30-12-1992 |
| | | | BR | 1100257 | A3 | 23-11-1999 |
| | | | MX | 9102003 | A1 | 01-12-1992 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 02 01 0824

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

24-07-2002

| Document brevet cité au rapport de recherche | Date de publication | Membre(s) de la famille de brevet(s) | Date de publication |
|---|---|---|---|
| EP 515240 A | 25-11-1992 | FR 2676054 A1 | 06-11-1992 |
| | | AT 158574 T | 15-10-1997 |
| | | AU 657321 B2 | 09-03-1995 |
| | | AU 1591892 A | 05-11-1992 |
| | | BR 9201655 A | 15-12-1992 |
| | | CA 2067924 A1 | 04-11-1992 |
| | | CS 9201328 A3 | 18-11-1992 |
| | | DE 69222352 D1 | 30-10-1997 |
| | | DE 69222352 T2 | 09-04-1998 |
| | | DK 515240 T3 | 11-05-1998 |
| | | EP 0515240 A1 | 25-11-1992 |
| | | ES 2109987 T3 | 01-02-1998 |
| | | FI 921950 A | 04-11-1992 |
| | | GR 3025277 T3 | 27-02-1998 |
| | | HU 65273 A2 | 02-05-1994 |
| | | IE 921365 A1 | 04-11-1992 |
| | | IL 101762 A | 16-10-1996 |
| | | JP 3108719 B2 | 13-11-2000 |
| | | JP 5140103 A | 08-06-1993 |
| | | KR 244063 B1 | 02-03-2000 |
| | | MX 9202026 A1 | 01-11-1992 |
| | | NO 921733 A ,B, | 04-11-1992 |
| | | NZ 242584 A | 27-04-1995 |
| | | RU 2089547 C1 | 10-09-1997 |
| | | US 5606065 A | 25-02-1997 |
| | | US 5411971 A | 02-05-1995 |
| | | ZA 9203176 A | 28-04-1993 |

EPO FORM P0460

Pour tout renseignement concernant cette annexe : voir Journal Officiel de l'Office européen des brevets, No.12/82